# EUROPEAN PATENT SPECIFICATION

(11) **EP 0 741 734 B1**
(45) Date of publication and mention of the grant of the patent: **04.04.2001**
(21) Application number: 95909329.5
(22) Date of filing: 27.01.1995
(51) Int. Cl.: C07F 5/02, G01N 33/53, G01N 33/72

(54) **PHENYLBORONIC ACID COMPLEXES**
PHENYLBORSAUREKOMPLEXE
COMPLEXES D'ACIDE PHENYLBORONIQUE

(30) Priority: 28.01.1994 US 188460; 28.01.1994 US 188531; 28.01.1994 US 188958; 28.01.1994 US 189176
(43) Date of publication of application: 13.11.1996
(73) Proprietor: PROLINX, INC., Woodinville, WA 98072 (US)
(72) Inventor: STOLOWITZ, Mark, L., Woodinville, WA 98072 (US)
(74) Representative: Molac, Béatrice
(86) International application number: US9501004
(87) International publication number: WO9520591

(56) References cited:
- WO-A-90/13818
- WO-A-92/08722
- US-A- 4 496 722

## Description

The present invention relates to the field of bioconjugate preparation, and more particularly, to a class of phenylboronic acid complexes useful for the conjugation of biological macromolecules, and the method of making and using such complexes.

Bioconjugation is a descriptive term for the joining of two or more different molecular species by chemical or biological means, in which at least one of the molecular species is a biological macromolecule. This includes conjugation of proteins, peptides, polysaccharides, lectins, hormones, nucleic acids, liposomes and cells, with each other or with any other molecular species that add useful properties, including radionuclides, toxins, haptens, inhibitors, fluorophores, ligands, etc. Immobilization of biological macromolecules is also considered a special case of bioconjugation in which the macromolecule is conjugated, either reversibly or irreversibly, to an insoluble support. Bioconjugation is utilized extensively in biochemical, immunochemical and molecular biological research. Applications of bioconjugation are numerous, and include affinity chromatography, affinity cytochemistry, histochemistry, pathological probe detection, diagnostics, signal amplification, immunoassay, hybridoma technology, blotting technology, bioaffinity sensors, gene probe detection, cross-linking reagents, affinity targeting, affinity perturbation, drug delivery, fusogenic reagents, immobilizing reagents, selective retrieval, selective elimination, flow cytometry and cytological probe detection. State of the art systems for preparing bioconjugates include Avidin-Biotin and Digoxigenin-anti-Digoxigenin systems.

Phenylboronic acids are known to interact with a wide range of polar molecules having the requisite functionalities. Complexes of varying stability, involving 1,2-diols, 1,3-diols, 1,2-hydroxy acids, 1,3-hydroxy acids, 1,2-hydroxylamines, 1,3-hydroxylamines, 1,2-diketones and 1,3-diketones, are known to form with either neutral phenylboronic acid or phenylboronate anion. Immobilized phenylboronic acid can be used as in chromatography to selectively retain from complex samples biological materials having the requisite functionalities. Many important biological molecules including carbohydrates, catecholamines, prostaglandins, ribonucleosides, and steroids contain the requisite functionalities, and can therefore be isolated in this way.

US 4,496,722 discloses compositions containing an organic boronic acid and one or more reporter groups. There is no disclosure of a phenylboronic acid moiety being bound to a phenylboronic acid complexing moiety as claimed in the present invention.

Phenylboronic acid, like boric acid, is a Lewis acid, and ionizes not by direct deprotonation, but by hydration to give the tetrahedral phenylboronate anion (p*K*_{*a*} = 8.86). Phenylboronic acid is three times as strong an acid as boric acid. Ionization of phenylboronic acid is an important factor in complexation, in that, upon ionization, boron changes from trigonal coordination (having average bond angles of 120° and average bond lengths of 1.37 Å) to the tetrahedrally coordinated anion (having average bond angles of 109° and average bond lengths of 1.48 Å). Development of systems utilizing phenylboronic acids with p*K*_{*a*} values below that of (3-aminophenyl)boronic acid (p*K*_{*a*} 8.75) (the most commonly commercially available derivative) would be desirable because it would enable the retention of a variety of biomolecules under physiological conditions (pH 7.2), thereby substantially extending the breath of compounds suitable for analysis by the method. Representative phenylboronic acids having a lower p*K*_{*a*} than (3-aminophenyl)boronic acid include the following:

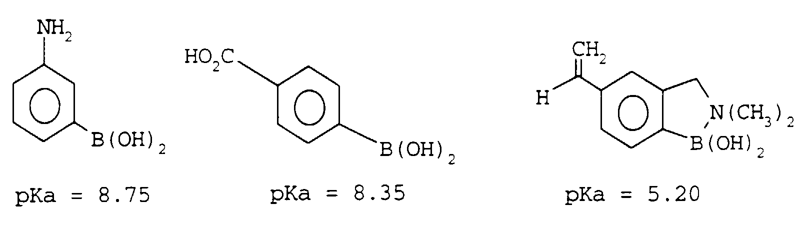

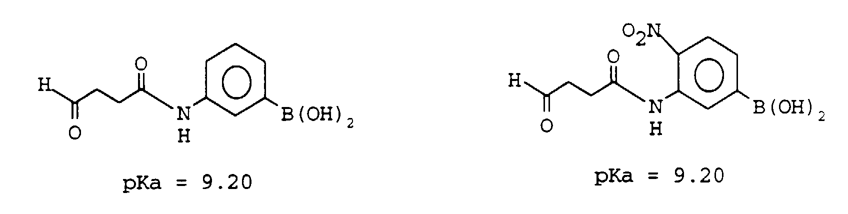

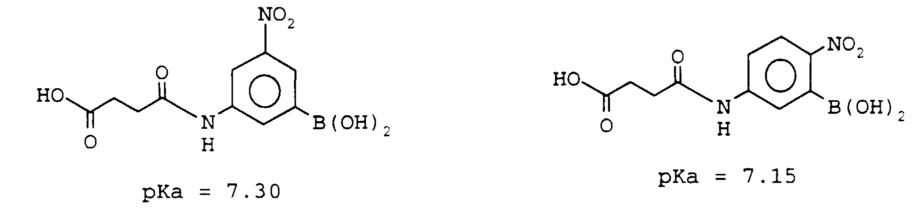

Compounds having cis or coaxial 1,2-diol and 1,3-diol functionalities, and particularly carbohydrates, are presently known to complex with immobilized phenylboronate anion, to form cyclic esters, only under alkaline aqueous conditions. Acidification of 1,2-diol and 1,3-diol complexes is known to release the diol containing species, presumably due to hydrolysis of the cyclic ester, which is induced by ring-strain associated with a five-membered cyclic boronic acid ester involving trigonally coordinated boron. Coplaner aromatic 1,3-diols, like 1,8-dihydroxynaphthalene, are known to complex even under acidic conditions due to the hydrolytic stability of six-membered cyclic boronic acid esters. Substituted phenols having pendant 1,3-hydroxylamide, 1,3-hydroxyamidine and 1,3-hydroxyoxime moieties are also known to complex reversibly with borate buffer, under alkaline aqueous conditions, in a manner analogous to that in which phenylboronic acids are known to complex.

Notwithstanding the substantial amount of research into bioconjugation, and the substantial amount of investment in this field, the selectivity of phenylboronic acid has not heretofore been exploited to enable the conjugation of biological macromolecules with one another or with other molecular species that add useful properties. Moreover, the use of immobilized complexing moities which can selectively complex with phenylboronic acid moieties is novel. This use is of particular interest, for example, where the phenylboronic acid moiety is linked to a biological macromolecule, such as an antibody, which can then be linked to the complexing moiety by exploiting the selectivity of the phenylboronic acid moiety for the complexing moiety.

As used herein the following terms have the following meanings:

*Bioactive species* refers to a compound preferably selected from, but not limited to, proteins, peptides, polysaccharides, hormones, nucleic acids, liposomes, cells, drugs, radionuclides, toxins, haptens, inhibitors, fluorophores, ligands and antibodies (e.g. monoclonal antibodies having specificity for epitopes on particular cell populations, e.g., particular hematopoetic cell populations, especially anti-CD34 antibodies). The bioactive species may also be a *solid phase support,* as defined below. Generally, bioactive species are collectively those species which confer biological activity or detection capabilities upon bioconjugate complexes. When the bioactive species is coupled to a semiconjugate or bioconjugate complex of the invention (e.g., corresponding to "BAS","BAS'", "BAS*" or "BAS*'" in formulae I-X, XIII, XV, XX, and XXI below), e.g., following reaction with an electrophilic or nucleophilic reactive moiety (e.g., corresponding to "R" in formulae XI, XII, XIV, XVI, or XVII below), it may optionally further comprise a residue of the aforementioned electrophilic or nucleophilic reactive moiety.

*Solid phase support* refers to a solid, insoluble surface or particle suitable for linkage to a phenylboronic acid complexing reagent or phenyl boronic acid reagent as herinafter defined (e.g. metal or plastic beads, e.g. optionally coated with carbohydrate or protein to facilitate binding to phenylboronic acid complexing reagents or phenylboronic acid reagents as hereinafter defined), for example suitable for use in an isolation or purification system or an assay system, e.g., a system utilizing a monoclonal antibody joined to a solid phase support in the form of a bioconjugate complex as hereinafter defined.

*Phenylboronic acid complexing reagent* refers to a reagent comprised of a phenylboronic acid complexing moiety and a reactive moiety suitable for appending a phenylboronic acid complexing moiety to a bioactive species or a solid phase support.

*Phenylboronic acid reagent* refers to a reagent comprised of a phenylboronic acid moiety and a reactive moiety suitable for appending a phenylboronic acid moiety to a bioactive species.

*Phenylboronic acid cross-linking reagent* refers to a reagent comprised of two phenylboronic acid moieties separated by a spacer.

*Phenylboronic acid complexing semiconjugate* refers to a bioactive species or solid phase support having a pendant phenylboronic acid complexing moiety which is derived from the reaction of the bioactive species or solid phase support with a phenylboronic acid complexing reagent.

*Phenylboronic acid semiconjugate* refers to a bioactive species having a pendant phenylboronic acid moiety which is derived from the reaction of a bioactive species with a phenylboronic acid reagent.

*Bioconjugate complex* refers to a bioconjugate linking two bioactive species (which may be the same or different) or a bioactive species and a solid phase support, wherein the linkage comprises at least one boron atom, e.g., at least one phenylboronic acid complex; for example, the product formed upon reaction of a phenylboronic acid complexing semiconjugate with a phenylboronic acid semiconjugate, or the product formed upon reaction of a phenylboronic acid complexing semiconjugate with a phenyl boronic acid cross-linking reagent.

Generally, the bioconjugate complexes of the invention are of Formulae I through X as set forth below, e.g., of general formula A

BAS-L-Bc-L'-(Bc'-L")ₙ-BAS' (A)

wherein BAS and BAS' are bioactive species (which may be the same or different); L, L', and L" are linkers (which may be the same or different; e.g., corresponding to groups Z, Z', Z*, Z*', Y and Y* in formulae I through X); Bc and Bc' are phenylboronic acid complexes (which may be the same or different) of formula D-E or E-D wherein D is a phenylboronic acid moiety (e.g. preferably derived from a derivative or analogue of phenylboronic acid) and E is a phenylboronic acid complexing moiety (e.g., preferably derived from an analogue of salicylic acid), and n is 0 or 1. Where BAS is a solid phase support, preferably Bc is E-D, and/or n=1, and/or BAS' is an antibody.

The present invention therefore provides a novel class of bioconjugate complexes derived from phenylboronic acid complexes, and the method of making and using such bioconjugate complexes. In the present invention, in the place of prior art Avidin-Biotin and Digoxigenin-anti-Digoxigenin systems, phenylboronic acid complexes are utilized to facilitate chemical conjugation of bioactive species without the use of intermediary biological macromolecules. Bioconjugate complexes linking two bioactive species wherein the boron is complexed with a nitrogen which itself is linked via a spacer moiety to a bioactive species are depicted, e.g., Formulae I through VI: 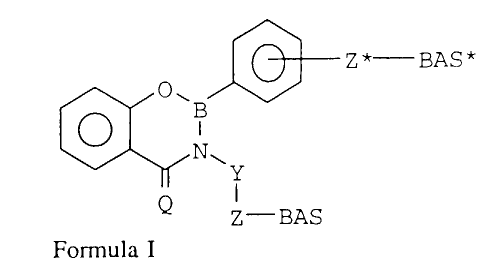 Bioconjugate complexes of Formula I are those wherein group Q is selected from either O, S, NH, N-alkyl, N-aryl and NCH₂-aryl wherein alkyl denotes a hydrocarbon moiety, e.g. of from 1 to 4 carbons in length, e.g., up to 6 carbons in length, wherein the chains may be branched, and aryl is selected from either an aromatic ring, a substituted aromatic ring and fused aromatic rings; wherein group Y is selected from either O, NH, CH₂, alkyl and aryl, wherein alkyl denotes a hydrocarbon moiety, e.g., of from 2 to 6 carbons in length and aryl is selected from either an aromatic ring, a substituted aromatic ring and fused aromatic rings; wherein groups Z and Z* (which may be the same or different) comprise a spacer selected from alkyl chains and and polyether (e.g., polyethyleneglycol) chains, of from 1 to 16 carbon equivalents in length, wherein the chain optionally may contain one or more intermediate amide and/or disulfide bonds; and wherein BAS and BAS* are the same or different bioactive species.

Bioconjugate complexes of Formula I are preferably those wherein group Q is selected from O, NH and NC₆H₅; wherein group Y is O and CH₂; wherein groups Z and Z^{*} are independently selected from (CH₂)ₙ, wherein n = 1 to 5, and (CH₂CH₂O)ₙ, wherein n' = 2 to 4; and/or wherein BAS and BAS* are two different bioactive species, e.g. wherein one is a solid phase support and the other an antibody. 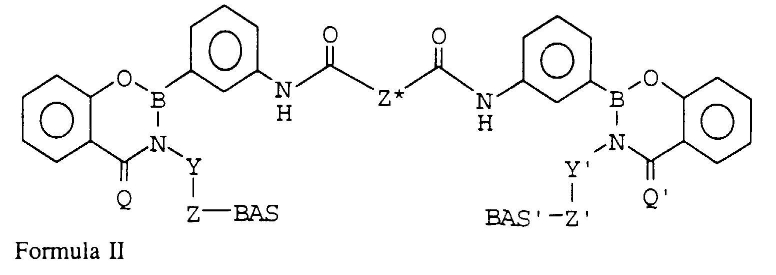 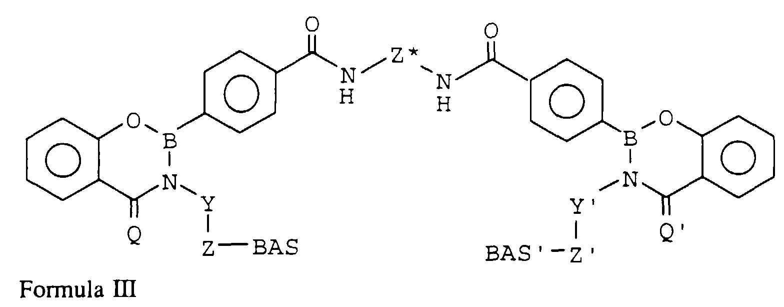 Bioconjugate complexes of Formulae II and III are those wherein groups Q and Q' are independently selected from either O, S, NH, N-alkyl, N-aryl and NCH₂-aryl wherein alkyl denotes a hydrocarbon moiety, e.g, of from 1 to 4 carbons in length, or up to 6 carbons in length, wherein the chains may be branched, and aryl is selected from either an aromatic ring, a substituted aromatic ring and fused aromatic rings; wherein groups Y and Y'are independently selected from either O, NH, CH₂, alkyl and aryl, wherein alkyl denotes a hydrocarbon moiety, e.g. of from 2 to 6 carbons in length, and aryl is selected from either an aromatic ring, a substituted aromatic ring and fused aromatic rings; wherein each of Z, Z', and Z* are spacers comprising an alkyl chain or polyether (e.g., polyethyleneglycol) chain of from 1 to 16 carbon equivalents in length, wherein the chain optionally may contain one or more intermediate amide and/or disulfide bonds; wherein group Z* is appended to two phenylboronic acid moieties; and wherein BAS and BAS' are the same or different bioactive species. Preferably when BAS and BAS' are the same, Q and Q', Y and Y', and Z and Z' are also the same.

Bioconjugate complexes of Formulae II and III are preferably those wherein groups Q and Q' are selected from O, NH and NC₆H₅; wherein groups Y and Y' are selected from O and CH₂; wherein Z, Z' and Z* may be the same or different and are selected from (CH₂)ₙ, wherein n = 1 to 5, and (CH₂CH₂O)ₙ., wherein n' = 2 to 4; and/or wherein the BAS groups are either the same bioactive species or one BAS is a solid phase support while the other is a bioactive species which is not a solid phase support, e.g., an antibody.

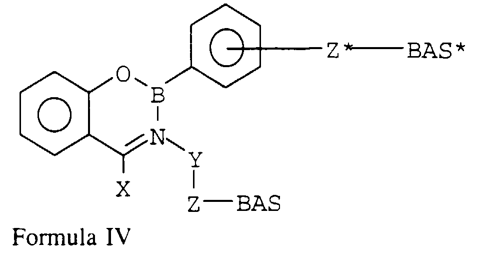

Bioconjugate complexes of Formula IV are those wherein group X is selected from H, CH₃ and C₆H₅; wherein group Y is selected from O, NH, CH₂, alkyl and aryl, wherein alkyl denotes a hydrocarbon moiety, e.g., from 2 to 6 carbons in length or up to 6 carbons in length, and aryl is selceted from either an aromatic ring, a substituted aromatic ring and fused aromatic rings; wherein groups Z and Z^{*} may be the same or different comprise a spacer selected from alkyl chains and polyether (e.g., polyethyleneglycol) chains, of from 1 to 16 carbon equivalents in length, wherein the chain optionally may contain one or more intermediate amide and/or disulfide bonds; and wherein groups BAS and BAS^{*} are the same or different bioactive species.

Bioconjugate complexes of Formula IV are preferably those wherein group X is selected from, but not limited to, either H and C₆H₅, wherein group Y is preferably selected from, but not limited to, either O and CH₂, wherein groups Z and Z^{*} may be the same or different and are preferably selected from, but not limited to, either (CH₂)ₙ, wherein n = 1 to 5, and (CH₂CH₂O)ₙ, wherein n' = 2 to 4; and/or wherein groups BAS and BAS^{*} are different bioactive species, preferably one being a solid phase support and the other a bioactive species which is not a solid phase support, e.g., an antibody.

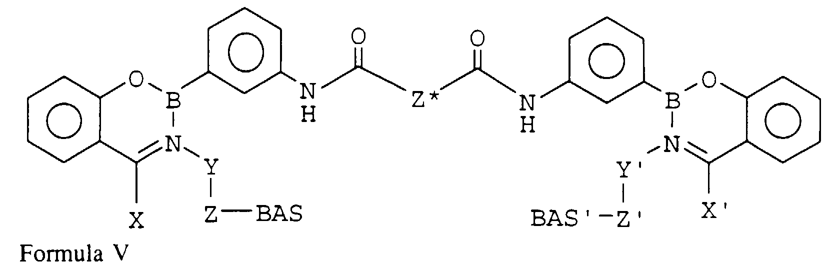

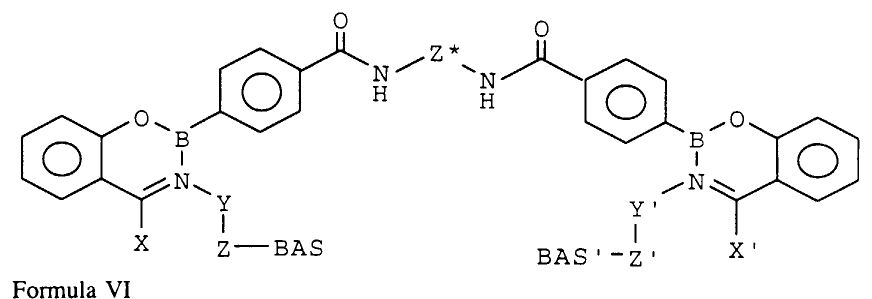

Bioconjugate complexes of Formulae V and VI are those wherein groups X and X' are independently selected from H, CH₃ and C₆H₅; wherein groups Y and Y' are independently selected from O, NH, CH₂, alkyl and aryl, wherein alkyl denotes a hydrocarbon moiety, e.g., from 2 to 6 carbon atoms in length, or up to 6 carbons in length, and aryl is selceted from an aromatic ring, a substituted aromatic ring and fused aromatic rings; wherein groups Z, Z' and Z* are spacers independently selected from alkyl chains and polyether (e.g., polyethyleneglycol) chains, of from 1 to 16 carbon equivalents in length, wherein the chain may optionally contain one or more intermediate amide and/or disulfide bonds; wherein group Z* is appended to two phenylboronic acid moieties; and wherein group BAS and BAS' are the same or different bioactive species. Preferably, when BAS and BAS' are the same, Z and Z', Y and Y', and X and X' are also the same.

Bioconjugate complexes of Formulae V and VI are preferably those wherein groups X and X' are independently selected from H and C₆H₅; wherein groups Y and Y' are independently selected from O and CH₂; wherein groups Z, Z' and Z* are independently selected from (CH₂)ₙ, wherein n = 1 to 5, and (CH₂CH₂O)ₙ, wherein n' = 2 to 4; and/or wherein groups BAS and BAS' are either the same or one is a solid phase support and the other is a bioactive species other than a solid phase support, e.g., an antibody.

Bioconjugate complexes of the invention also comprise those complexes where at least one of the bioactive species is linked to the benzene ring of the phenylboronic acid complexing moiety, e.g. as in Formulae VII through X.

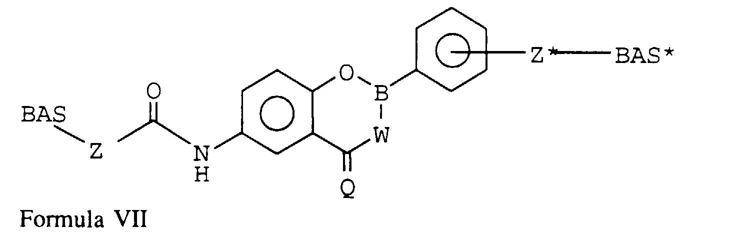

Bioconjugates of Formula VII are those wherein group W is selected from O, NH, N-alkyl, NC₆H₅, N-aryl, NCH₂-aryl, NCH₂CH₂OH, NCOCH₂CH₂OH, NOH, NO-alkyl and NOCH₂-aryl, wherein alkyl denotes a hydrocarbon moiety, e.g. of from 1 to 4 carbons in length, or up to 6 carbons in length, wherein the chains may be linear or branched, and aryl is selected from an aromatic ring, a substituted aromatic ring and fused aromatic rings; wherein group Q is selected from either O, S, NH, N-alkyl, N-aryl and NCH₂-aryl, wherein alkyl and aryl are as was previously defined; wherein groups Z and Z* are the same or different and comprise a spacer selected from alkyl chains and polyether (e.g., polyethyleneglycol) chains, of from 1 to 16 carbon equivalents in length, wherein the chain may optionally contain one or more intermediate amide and disulfide bonds; and wherein groups BAS and BAS* are the same or different bioactive species.

Bioconjugate complexes of Formula VII are preferably those wherein group W is selected from O, NH, NCH₃, NC₆H₅, NCH₂CH₂OH, NCOCH₂CH₂OH, NOH and NOCH₃; wherein group Q is O; wherein groups Z and Z* are independently selected from (CH₂)ₙ, wherein n = 1 to 5, and (CH₂CH₂O)ₙ, wherein n' = 2 to 4; and/or wherein BAS and BAS^{*} are different bioactive species, e.g., wherein one is a solid phase support and the other is a bioactive species other than a solid phase support, e.g., an antibody.

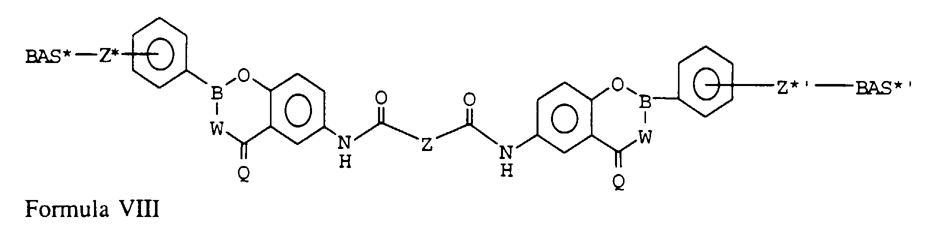

Bioconjugate complexes of Formula VIII are those wherein group W is selected from O, NH, N-alkyl, NC₆H₅, N-aryl, NCH₂-aryl, NCH₂CH₂OH, NCOCH₂CH₂OH, NOH, NO-alkyl and NOCH₂-aryl, wherein alkyl denotes a hydrocarbon moiety, e.g. of from 1 to 4 carbons in length, or up to 6 carbons in length, wherein the chains may be branched, wherein aryl is selected from either an aromatic ring, a substituted aromatic ring and fused aromatic rings; wherein group Q is selected from O, S, NH, N-alkyl, N-aryl and NCH₂-aryl, wherein alkyl and aryl are. as was previously defined; wherein groups Z, Z*, and Z*' are the same or different and comprise a spacer selected from alkyl chains and polyether (e.g., polyethyleneglycol) chains, of from 1 to 16 carbon equivalents in length, wherein the chain may optionally contain one or more intermediate amide and disulfide bonds, wherein group Z is appended to two phenylboronic acid complexing moieties, and wherein groups BAS* and BAS*' are the same or different bioactive species.

Bioconjugate complexes of Formula VIII are preferably those wherein group W is selected from O, NH, NCH₃, NC₆H₅, NCH₂CH₂OH, NCOCH₂CH₂OH, NOH and NOCH₃; wherein group Q is preferably O, and wherein groups Z, Z*, and Z*' are the same or different and preferably selected from (CH₂)ₙ, wherein n = 1 to 5, and (CH₂CH₂O)ₙ, wherein n' = 2 to 4; and/or wherein BAS* and BAS*' are either the same or one is a solid phase support and the other is a bioactive species other than a solid phase support, e.g., an antibody.

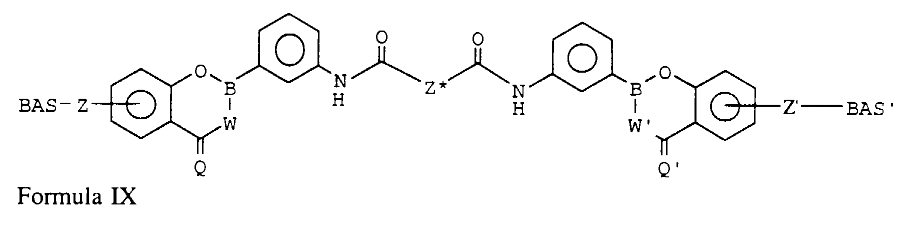

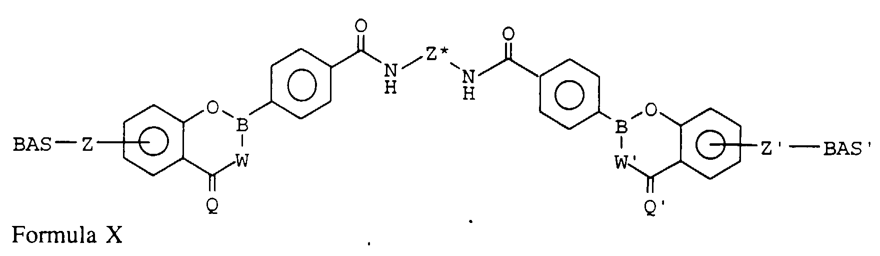

Bioconjugate complexes of Formulae IX and X are those wherein groups W and W' are independently selected from O, NH, N-alkyl, NC₆H₅, N-aryl, NCH₂-aryl, NCH₂CH₂OH, NCOCH₂CH₂OH, NOH, NO-alkyl and NOCH₂-aryl, wherein alkyl denotes a hydrocarbon moiety, e.g. of from 1 to 4 carbons in length, e.g., up to 6 carbon atoms in length, wherein the chains may optionally be branched, wherein aryl is selected from an aromatic ring, a substituted aromatic ring and fused aromatic rings; wherein groups Q and Q' are independently selected from O, S, NH, N-alkyl, N-aryl and NCH₂-aryl, wherein alkyl and aryl are as was previously defined; wherein groups Z, Z' and Z* are spacers independently selected from alkyl chains and polyether (e.g., polyethyleneglycol) chains, of from 1 to 16 carbon equivalents in length, wherein the chain may optionally contain one or more intermediate amide and disulfide bonds, wherein group Z* is appended to two phenylboronic acid moieties;and wherein BAS and BAS' are the same or different bioactive species. Where BAS and BAS' are the same, preferably, W and W', Q and Q', and Z and Z' are also the same.

Bioconjugate complexes of Formula IX and X are preferably those wherein groups W and W' are independently selected from O, NH, NCH₃, NC₆H₅, NCH₂CH₂OH, NCOCH₂CH₂OH, NOH and NOCH₃; wherein group Q is preferably O; wherein groups Z, Z' and Z^{*} are the same or different and are preferably selected from (CH₂)ₙ, wherein n = 1 to 5, and (CH₂CH₂O)ₙ., wherein n' = 2 to 4; and/or wherein BAS and BAS' are either the same bioactive species, or where one is a solid phase support, the other is a bioactive species other than a solid phase support, e.g., an antibody.

Bioconjugate complexes having a single phenylboronic acid complex, e.g., as in Formulae I, IV and VII, are preferably employed to conjugate two different bioactive species, for example, to conjugate an enzyme with and antibody for use in an ELISA assay, to conjugate a nucleic acid probe with a fluorophore to facilitate detection of a genomic sequence, to conjugate a toxin to a monoclonal antibody for use in targeted drug delivery. For example, such bioconjugate complexes may be used to conjugate an antibody (e.g., a monoclonal antibody capable of binding selectively to an epitope on a hematopoietic cell, e.g. anti-CD 34 antibody) to a solid phase (e.g. a bead of metal or plastic optionally coated with an organic substance e.g., carbohydrate or protein), e.g. where the antibody is BAS and the solid surface is BAS*, or where the antibody in BAS* and the the solid surface is BAS.

Bioconjugate complexes having two phenylboronic acid complexes, e.g., as in Formulae II, III, V, VI, VIII, IX and X are preferably employed to conjugate identical bioactive species by cross-linking bioactive species having pendant phenylboronic acid or phenylboronic acid complexing moities into macromolecular aggregates. Aggregates of this type involving enzymes are useful for increasing detection limits in ELISA and related assays by substantially increasing the effective concentration of enzyme available for conversion of colorless substrate into detectable product. Similarly, fluorophore labeled proteins having pendant phenylboronic acid complexing moieties my be aggregated in this manner to improve their visual or spectrophotometric detection. Aggregates having excess pendant phenylboronic acid moities may be further conjugated with other bioactive species having pendant phenylboronic acid complexing moieties (phenylboronic acid complexing semiconjugates). This general approach is analogous to the preparation of sandwich type assays involving the Avidin Biotin system.

Phenylboronic acid cross-linking reagents may also be employed, by reaction in large excess, and subsequent removal of excess reagent, to convert bioactive species having pendant phenylboronic acid complexing moities (phenylboronic acid complexing semiconjugates) into bioactive species having pendant phenylboronic acid moities (phenylboronic acid semiconjugates), and vice versa.

Normally, the spacer moiety joining the bioactive species to the phenyl of the phenylboronic acid moiety (e.g., Z* or Z*' in formula I, VII, or VIII, or Z* in XIV or XV infra) is present. However, in some cases, the bioactive species may have a configuration permitting linkage directly to the phenyl via an electrophilic or nucleophilic moiety (R) in formula XIV without the need for a spacer. Thus the invention further relates to and encompasses compounds of formula I, VII. VIII. XIV. or XV where Z* or Z*' is not present.

Bioconjugate complexes of Formula I through X are prepared in either buffered aqueous solutions, organic solvents and aqueous solutions containing organic solvents. The complex is formed within a few minutes at room temperature. Preparation of the bioconjugate complex is insensitive to significant variations in ionic strength, temperature, and the presence of chaotropic agents (protein denaturants), which are incompatible with prior art systems wherein the structure of a biological macromolecule must be maintained to preserve requisite binding properties. In most instances, the constraints governing the formation of bioconjugate complexes, by the system described herein, are limited to the selection of an appropriate pH and whatever additional limitations are imposed by the conditions required to maintain viability of the bioactive species.

The present invention further provides reagents suitable for the modification of a bioactive species for the purpose of incorporating a phenylboronic acid complexing moiety for subsequent conjugation to a different bioactive species having pendant phenylboronic acid moieties e.g. in the preparation of a bioconjugate of formula VII, VIII, IX or X: 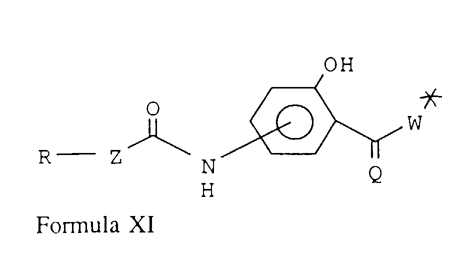 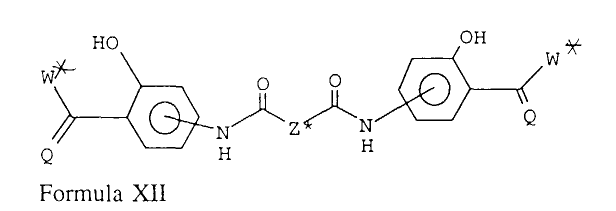 wherein the groups Q, Z and Z* are as defined for any of formulae VII, VIII, IX or X; wherein group W* is selected from H, OH, NH₂, NHCH₃, NHOH and NHOCH₃; wherein group Q is selected from O, S and NH; and/or Z and Z* are spacers independently selected from alkyl and polyethyleneglycol chains, of from 1 to 16 carbon equivalents in length, wherein the chain may contain intermediate amide and disulfide bonds. Group R in formula XI is a reactive electrophilic or nucleophilic moiety suitable for reaction of the phenylboronic acid complexing reagent with a *bioactive species.*

Reagents of Formulae XI and XII are preferably those wherein group W* is selected from either OH, NHOH and NHOCH₃, and/or group Q is O. Group Z and Z* are preferably selected (CH₂)ₙ, wherein n = 1 to 5, or (CH₂CH₂O)ₙ, wherein n = 2 to 4.

Reagents of Formula XI are preferably those wherein group R is selected from, but is not limited to, amino, hydrazide, N-hydroxysuccinimidyl ester, *N*-hydroxysulfosuccinimidyl ester, isothiocyanate, bromoacetamide, iodoacetamide, maleimide and thiol moieties. Reaction of a reagent of General Formula XI with a *bioactive species* affords a semi-conjugate having pendant phenylboronic acid complexing moieties (one or more) of Formula XIII, wherein the symbol labeled BAS represents the *bioactive species,* group W*, is as above defined, and groups Q and Z are as were previously defined for formula VII 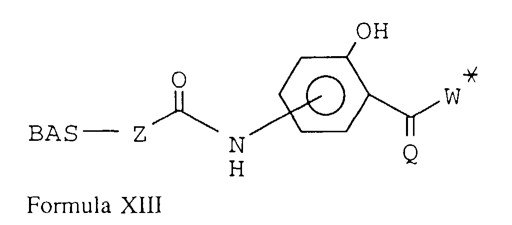 Similarly, phenylboronic acid reagents [e.g. of formula XIV: 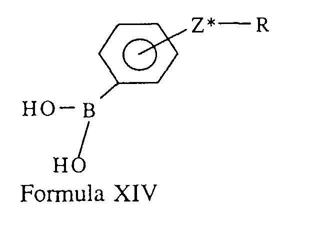 wherein Z* is a spacer as defined for any of formulae I, IV, VII or VIII and R is a reactive electrophilic or nucleophilic moiety as defined for formula XI] may be appended to a *bioactive species* to afford a semiconjugate having pendant phenylboronic acid moieties (one or more) of Formula XV 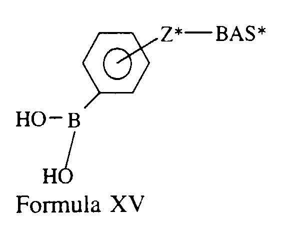 wherein the symbol labeled BAS* represents a *bioactive species,* e.g. the same or different from the *bioactive species* labeled BAS, and group Z^{*} is as defined for formula VII.

We note here that semiconjugates of Formula XV and other phenyboronic acid reagents and semiconjugates described herein involve either the tetrahedral phenylboronate anion under alkaline conditions, or triganol phenylboronic acid under neutral or acidic conditions, e.g., 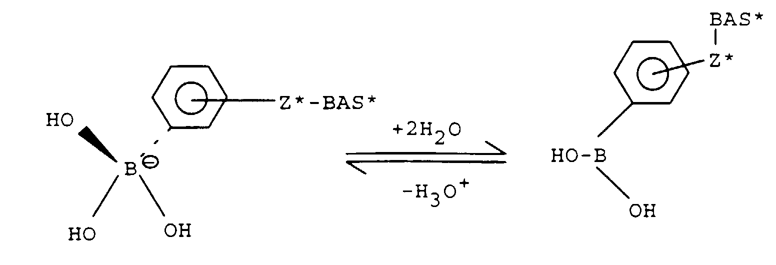 and both forms are intended to be encompassed by the invention herein.

Phenylboronic acid crosslinking reagents of formula XVI: 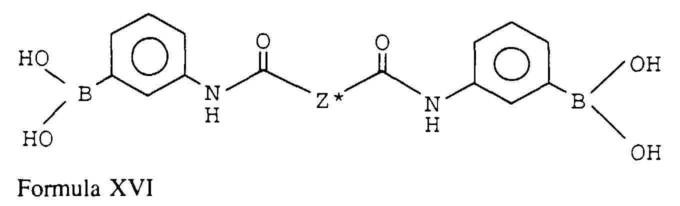 wherein Z^{*} is a linker as defined in any of formula II, V, or IX; or formula XVII: 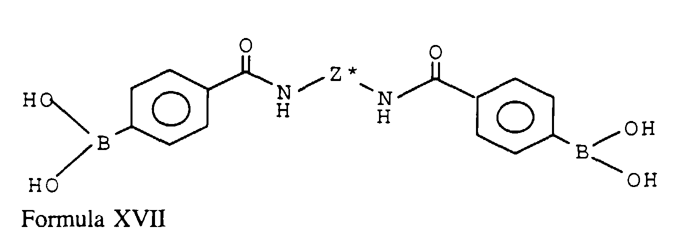 wherein Z^{*} is a linker as defined in any of formulae III, VI or X are also provided.

A semiconjugate of Formula XIII, prepared from *bioactive species* BAS and having pendent phenylboronic acid complexing moities, may be complexed with a semiconjugate of Formula XV, prepared from a second *bioactive species* BAS^{*} and having pendant phenylboronic acid moities, to afford a bioconjugate of Formula VII. In this manner, biological macromolecules may be conjugated to one another or with other functionalities which impart useful properties.

Similarly, a reagent of Formula XII may be complexed with a semiconjugate of Formula XIV, prepared from a *bioactive species* BAS^{*}, to afford a bioconjugate of Formula VIII. In this manner, two or more identical *bioactive species* BAS^{*}, may be conjugated to one another. This process may be exploited to prepare enzyme aggregates, which are useful for high sensitivity detection during ELISA.

The bioconjugate are prepared in buffered aqueous or aqueous/organic solutions. The bioconjugates are formed within a few minutes at room temperature. The stability of a given bioconjugate at a given pH is determined by substituent groups W and Q. Bioconjugates of Formulae VII and VIII, wherein both groups W and Q are O, are stable in acidic aqueous solutions of approximate pH less than 4.5. Bioconjugates of Formulae VII and VIII, wherein group W is selected from either NH and NCH₃, and wherein group Q is selected from either O and S, are stable in buffered alkaline aqueous solutions over the approximate pH range 8.5 to 11.5. Similarly, bioconjugates of Formulae VII and VIII wherein both groups W and Q are NH, are stable in buffered alkaline aqueous solutions over the approximate pH range 8.5 to 11.5. Bioconjugates of Formulae VII and VIII, wherein group W is selected from either NOH and NOCH₃, and group Q is selected from either O and S, are stable in buffered aqueous solutions over the broad approximate pH range 2.5 to 11.5.

The bioconjugation reaction (phenylboronic acid complexation) is insensitive to significant variations in ionic strength, temperature, the presence of organic solvents, and the presence of chaotropic agents (protein denaturants), which are incompatible with prior art systems wherein the structure of a biological macromolecule must be maintained to preserve requisite binding properties. In most instances, the constraints governing the formation of bioconjugates, by the system herein described, are limited to those imposed by the conditions required to maintain viability of the bioactive species.

In a further embodiment, the invention provides phenylboronic acid reagents, e.g., reagents suitable for the modification of a bioactive species for the purpose of incorporating a phenylboronic acid complexing moiety e.g. in the preparation of a bioconjugate complex of formula I, II, III, IV, V or VI, e.g. reagents selected from either of general formula XVIII or formula XIX.

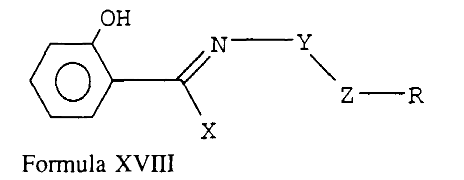

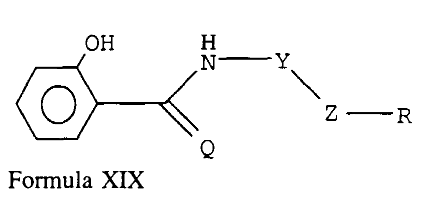

For formula XVIII, X, Y and Z are as defined for formula IV, V or IV. Preferably reagents of formula XVIII are those wherein X is selected from either H, CH₃ and C₆H₅, and/or wherein Y is selected from either O and CH₂. For formula XIX, Q, Y and Z are as defined for formulae I, II or III. Preferably, reagents of formula XIX are those wherein group Y is selected from either O and CH₂, and/or Q is O. In both formulae XVIII and XIX, group R is a reactive electrophilic or nucleophilic moiety suitable for reaction of the phenylboronic acid complexing reagent with a bioactive species.

Reagents of general formula XVIII are most preferably those wherein group X is selected from either H and CH₃, and group Y is O. Reagents of general formula XVI are preferably those wherein group Y is O. In both formulae XVIII and XIX, group Z is preferably selected from either an alkyl chain or polyether (e.g. polyethyleneglycol) chain, of from 1 to 16 carbon equivalents, preferably 2 to 12 carbon equivalents, in length, and which may contain intermediate amide and/or disulfide functionalities, and is preferably (CH₂)ₙ, wherein n = 2 to 6, or (CH₂CH₂O)ₙ, wherein n = 2 to 4. In both formulae XVIII and XIX, group R is preferably selected from, but is not limited to, either hydrazide, isothiocyanate, *N*-hydroxysuccinimidyl ester, *N*-hydroxysulfosuccinimidyl ester, imidate ester, 2,2,2-tri-fluoroethanesulfonyl, bromoacetamide, iodoacetamide, maleimide and 2-cyanoethyl-*N*,*N*-diisopropylphosphoramidite ester moieties.

Reagents of formula XVIII or XIX are reacted with a bioactive species BAS (or BAS*) or form the corresponding semiconjugate of formula XX and XXI respectively: 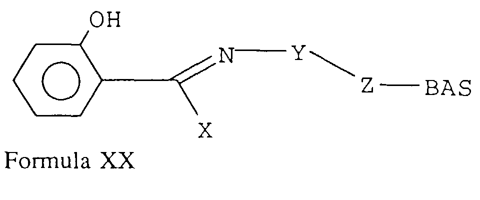 wherein X, Y, and Z are as defined for formula XVIII 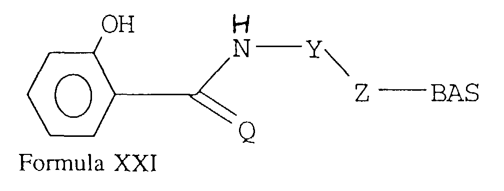 wherein Y, Z, and Q arc as defined for formula XIX

The invention thus describes :
1. Bioconjugate complexes as previously defined, e.g. of any of formulae I through X;
2. Phenylboronic acid complexing semiconjugate as previously defined, e.g. of any of formulae XIII, XX and XXI;
3. Phenylboronic acid semiconjugates as previously defined, of formula XV;
4. Phenylboronic acid complexing reagents as previously defined , e.g. of any of formulae XI, XVIII and XIX;
5. Phenylboronic acid reagents as previously defined, e.g. of formula XIV; and
6. Phenylboronic acid cross-linking reagents as previously defined, e.g. of formulae XII, XVI and XVII.

The invention further provides the use of the foregoing compounds in any of the previously listed applications for bioconjugation, particularly in an antibody-based assay or purification system; and the use of the foregoing semiconjugates, crosslinkers, and reagents (e.g., of formulae XI through XXI) in the production of bioconjugate complexes (e.g., of formulae I though X).

In a particular embodiment, the invention provides a kit or system for isolating or purifying cells, e.g. hematopoietic cells, e.g. CD 34⁺ cells, comprising a bioconjugate complex (according to any one of formula I through X) linking a first and second bioactive species wherein the first bioactive species is a solid phase support, e.g. a bead of metal or plastic (optionally coated with carbohydrate, protein or other organic material to facilitate reactivity and binding to a reagent of the invention, e.g. at R in formulae XI, XIV, XVIII or XIX) and the second bioactive species is an antibody, e.g. an antibody capable of recognising and binding to an epitope present on a particular cell population, e.g. CD 34⁺ cells; as well as further providing a method for isolating or purifying cells as described, comprising the steps of contacting a medium containing the desired cells with such a bioconjugate complex of the invention having a second bioactive species as described selective for the desired cell population, separating the cells thus selected from the medium, and optionally separating the selected cells from the bioconjugate complex.

Bioconjugate complexes of formula I, IV or VII are prepared by a three-step process in which:
(1) A phenylboronic acid complexing reagent e.g. of formula XI, XVIII, or XIX, preferably derived from salicylic acid, aminosalicylic acid, or dithiosalicylic acid, is condensed with a bioactive species to prepare a phenylboronic acid complexing semiconjugate;
(2) A phenylboronic acid reagent of e.g. derived from a compound preferably selected from, but not limited to, either (3-aminophenyl)boronic acid and (4- carboxyphenyl)boronic acid, e.g. of formula XIV is condensed with a bioactive species to prepare a phenylboronic acid semiconjugate;
(3) The phenylboronic acid complexing semiconjugate, prepared as described in (1) above, and the phenylboronic acid semiconjugate, prepared as described in (2) above, are reacted with one another to afford a bioconjugate complex e.g. of formula I, IV, or VII.

Bioconjugate complexes of formulae II, V, or IX are prepared by a two-step process in which:
(1) A phenylboronic acid complexing reagent of formula XI, XVIII or XIX is condensed with a bioactive species to prepare phenylboronic acid complexing semiconjugates;
(2) The phenylboronic acid complexing semiconjugates, prepared as described in (1) above, are reacted with a phenylboronic acid crosslinking reagent preferably derived from (3-aminophenyl)boronic acid, e.g. of formula XVI.

Bioconjugate complexes of formulae III, VI or X are prepared by a two-step process in which:
(1) A phenylboronic acid complexing reagent of formula XI, XVIII or XIX is reacted with a bioactive species to prepare phenylboronic acid complexing semiconjugates;
(2) The phenylboronic acid complexing semiconjugates, prepared as described in (1) above, are reacted with a phenylboronic acid crosslinking reagent preferably derived from (4-carboxyphenyl)boronic acid, e.g. of formula XVII, to afford the desired complex.

Bioconjugate complexes of formula VIII are prepared by a two step process in which:
(1) The phenylboronic acid reagent of formula XIV is reacted with a bioactive species to prepare the semiconjugate of formula XV.;
(2) The semiconjugate of formula XV is reated with a phenylboronic acid crosslinking reagent preferably derived from salicylic acid, aminosalicylic acid, or dithiosalicylic acid, of formula XII, to afford the desired complex.

Reagents of formula XIV are derived from phenylboronic acid derivatives and analogues, e.g., compounds preferably selected from, but not limited to, (3-aminophenyl)boronic acid, (4-carboxy-phenyl)boronic acid and *N*-(6-nitro-3-dihydroxyborylphenyl)succinamic acid, (3-isothiocyanatophenyl)boronic acid, (5-carboxy-3-isothiocyanatophenyl)boronic acid, (3-iodoacetamidophenyl)boronic acid, (3-maleimidophenyl)boronic acid, (3- dihydroxyborylphenyl)succinamic acid succinimidyl ester and (3-dihydroxyborylphenyl)succinamic acid hydrazide, which can be commercially obtained or synthesized by the methods described or analogously to the methods described, e.g., in Linder, K. E., Wen, M. D., Nowotnik, D. P., Malley, M. F., Gougoutas, J. Z., Nunn, A. D. and Eckelman, W.C. (1991) *Bioconjugate Chem.,* **2**, 160-170, and Linder, K. E., Wen, M. D., Nowotnik, D. P., Ramalingam, K., Sharkey, R. M., Yost, F., Narra, R. K., Nunn, A. D. and Eckelman, W.C. (1991) *Bioconjugate Chem.,* **2**, 407-415.

Phenylboronic acid reagents of Formula XVI are prepared by condensation of (3-aminophenyl)boronic acid with an activated dicarboxylic acid preferably selected from, but not limited to, either succinyl chloride, adipoyl chloride, adiptic acid diisobutylcarbonate, suberoyl chloride, 3,3'-dithiopropionyl chloride and 3,6,9-trioxaundecanedioyl chloride and 3,6,9-trioxa-undecanedioic acid diisobutylcarbonate, analogously to the methods described in Burnett, T. J., Peebles, H. C. and Hageman, J. H. (1980) *Biochem. Biophys. Research Commun.,* **96**, 157-162.

Phenylboronic acid reagents of Formula XVII are prepared by activation of (4-carboxyphenyl)boronic acid with *N,N*-dicyclohexylcarbodiimide, followed by condensation with a diamine, preferably selected from, but not limited to, either 1,4-butanediamine, 1,6-hexanediamine and 2,2'-dithiodiaminoethane (H₂NCH₂CH₂SSCH₂CH₂NH₂).

The bioconjugate complexes are prepared in buffered aqueous solutions preferably selected from, but not limited to, acetate, citrate, phosphate and carbonate buffers. Borate and Tris buffers should be avoided due to their potential for complexation with phenylboronic acid complexing moities and phenylboronic acid moieties, respectively. The bioconjugate complex is formed within 1 to 15 minutes at room temperature. The reaction is insensitive to variations in ionic strength over the range 0.01 to 2 molar. Stability of the complex increases with increasing temperature, limited only by the volatility of the buffer. Addition of organic solvents including acetonitrile, methanol, ethanol, isopropanol, butanol, *N,N-* dimethylformamide and dimethylsulfoxide serves to further stabilize bioconjugates. Chaotropic reagents (protein denaturants) including urea, guanidine hydrochloride and formamide also serve to further stabilize bioconjugates, if and when the bioactive species is tolerant of their presence. Bioconjugate complexes may be purified by desalting, dialysis, size exclusion chromatograpihy and electrophoresis. Bioconjugate complexes are stable upon removal of water, and can be lyophilized for storage.

Ionization of phenylboronic acid is an important factor in bioconjugate complex formation, in that, upon ionization boron changes from trigonal coordination (having average bond angles of 120° and average bond lengths of 1.37 Å) to the tetrahedrally coordinated anion (having average bond angles of 109° and average bond lengths of 1.48 Å). Phenylboronic acids vary in p*K*_{*a*} between approximately 5.2 and 9.2. Bioconjugate complexes of General Formulas I, III, IV, VI, VII, VIII and X derived from (4-carboxyphenyl)boronic acid, have approximate p*K*_{*a*} values in the range 6.5 to 7.5. Bioconjugate complexes of General Formulas I, II, IV, V, VI, VII and IX derived from (3-aminophenyl)boronic acid), have approximate p*K*_{*a*} values in the range 8.0 to 9.0. Complexes of formulae I, II, IV, V, VII and IX derived from (3-amino-2-nitrophenyl)boronic acid, (3-amino-5-nitrophenyl)boronic acid, or (3-amino-6-nitrophenyl)boronic acid have intermediate p*K*_{*a*} values. As a general rule, the p*K*_{*a*} of the complex of General Formulas I through X is approximately one pH unit below that of the phenylboronic acid from which the complex was prepared.

Bioconjugate complexes of formulae I, II and III (e.g. wherein Q and Q' are preferably selected from either O, S and NH; and/or wherein Y and Y' are preferably selected from either O and NH) wherein the phenylboronic acid moiety is derived from (3- aminophenyl)boronic acid, are stable in buffered alkaline aqueous solutions over the approximate pH range 8.5 to 11.5. Similarly, bioconjugate complexes of General Formulas IV, V and VI (e.g., wherein groups X and X' are preferably selected from either H, CH₂ and C₆H₅ and/or wherein groups Y and Y' are preferably selected from either O and NH) wherein the phenylboronic acid moiety is derived from (3- aminophenyl)boronic acid, are stable in buffered alkaline aqueous solutions over the approximate pH range 8.5 to 11.5. This range of pH stability results from the requierment that only the phenylboronate anion affords a stable complex. Nevertheless, above pH 11.5 the complex is unstable, due to base catalyzed hydrolysis. Bioconjugate complexes which exhibit stability only under alkaline conditions are useful for reversible conjugation, whereby the bioconjugate complex may be disassociated by appropriate adjustment of the pH.

Bioconjugate complexes of formulae I, II and III (e.g., wherein groups Q, Q', Y and Y' are preferably O) wherein the phenylboronic acid moiety is derived from either (3- amino-phenyl)boronic acid and (4-carboxyphenyl)boronic acid, constitute a special case in which complexes are stable in buffered aqueous solutions over the broad approximate pH range 2.5 to 11.5. This wide range of pH stability is thought to result from the presence of a coplanar 1,3-diol complexing moiety associated with the enol form of the 2-hydroxybenzohydroxamic acid moiety. Alternatively, pH stability may result from the low effective p*K*_{*a*} of the phenylboronic acid in the complex which results from reaction of a hydroxamic acid anion (CON⁻OH) with phenylboronic acid forming a dative bond which fills the outter electron shell of the boron atom. Bioconjugate complexes of this type form in an essentially irreversible manner, as they may only be disassociated by adjustment of the pH to above 11.5, or below 2.5, or by competitive dissociation with borate buffer.

Phenylboronic acid complexing semiconjugates wherein the bioactive species is a protein, may be characterized with respect to the number of pendant phenylboronic acid complexing moieties incorporated (degree of substitution) per protein molecule. Semiconjugates may be treated with an excess of a fluorescent phenylboronic acid reagent, in a buffered aqueous solution of appropriate pH, to afford a bioconjugate complex of formula I, IV, or VII wherein previously defined, and wherein BAS* to a fluorescent moiety. Similarly, phenylboronic acid semiconjugates may be characterized by reaction with an excess of a fluorescent phenylboronic acid complexing reagent, in a buffered aqueous solution of appropriate pH, to afford bioconjugate complexes of formulae I, IV, or VII wherein BAS* is a fluorescent moiety.

Suitable fluorescent moieties are preferably selected from, but not limited to, either fluorescein, rhodamine X, tetramethylrhodamine, Texas Red, phycoerythrin and allophycocyanin. After removal of the excess reagent by desalting, dialysis or size exclusion chromatography, the bioconjugate complex is subjected to spectroscopic analysis, and the number of phenylboronic acid complexing moieties or phenylboronic acid moities, calculated by comparing the ratio of the absorption at 280 nm, which denotes the total protein concentration, to the absorption at a wavelength characteristic of the fluorophore (|ₘₐₓ). Semiconjugates derived from other high molecular weight bioactive species which are suitable for purification by desalting, dialysis or size exclusion chromatography may be characterized in an analagous manner.

### Example I: Preparation of An Amine Reactive Reagent of Formula XIV N-(3-Dihydroxyborylphenyl)succinamic Acid, Succinimidyl Ester

Succinic anhydride (5.00 grams, 0.05 mole) and (3-aminophenyl)boronic acid (7.75 grams, 0.05 mole) are dissolved in anhydrous pyridine (40ml), and then allowed to stand overnight at room temperature. Water (20 ml) is added and the resulting solution allowed to stand for 1 hour. The product is then concentrated on a rotary evaporator at 85-90° C. The resulting aqueous solution is frozen in a dry-ice-acetone-slurry and lyophilized overnight. The lyophilized product is dissolved in water (50 ml) and acidified with concentrated HCI to approximately pH 1.0. The acidified solution is cooled in an ice bath for 1 hour, and the precipitate collected by filtration. The precipitate is recrystallized from boiling water (200 ml) and dried overnight in *vacuo* over NaOH pellets, to afford 8.60 grams (70% yield) of *N*-(3-dihydroxyborylphenyl)succinamic acid. Homogeneous by TLC (CHCl₃/CH₃OH/CH₃COOH; 60:35:5), R_{f} = 0.5. Melting point 186-188°C. The structure was confirmed by 300 MHz, ¹H NMR spectrometry in d₆-DMSO.

*N*-(3-dihydroxyborylphenyl)succinamic acid (16.0 grams, 0.063 mole) is dissolved in dry DMF (80 ml). To the solution is added *N,N*-dicyclohexylcarbodiimide (14.3 grams, 0.069 mole) followed by *N*-hydroxysuccinimide (8.05 grams, 0.070 mole). The reaction is stirred overnight at room temperature. *N,N*-Dicyclohexylurea is filtered from the solution, and the filtrate extracted with ethyl acetate (200 ml). The extract is washed with water (3 X 400 ml) and saturated NaCI (400 ml). The water wash was back-extracted with ethyl acetate (200 ml), the extracts combined, dried over anhydrous Na₂SO₄, and concentrated on a rotary evaporator to afford 12.5 grams (57% yield) of *N*-(3-dihydroxyboryl-phenyl)succinamic acid, succinimidyl ester. Purity estimated at 98% by TLC (CHCl₃/CH₃OH/CH₃COOH; 85:10:5), R_{f} = 0.7. The structure was confirmed by 300 MHz, ¹H NMR spectrometry in d₆ DMSO.

### Example II: Applications of An Amine Reactive Reagent of General Formula XIV

Proteins may be modified with amine reactive phenylboronic acid reagents of Formula XIV by reaction with the side-chain e-amino groups of lysine residues, to afford semiconjugates having pendant phenylboronic acid moieties covalently affixed to the protein through stable amide bonds. *N,N*-Dimethylformamide and dimethylsulfoxide are the solvents of choice. Mildly alkaline aqueous buffers, in the pH range 7.8 to 8.8, and preferably 100mM bicarbonate buffer, pH 8.2, should be employed so as to insure that the amino group is unprotonated, while minimizing hydrolysis of the *N*-hydroxysuccinimidyl ester. Activated N-hydroxysuccimidyl esters have been observed to interact with phenylboronic acids in alkaline aqueous solutions resulting in a significant reduction in their reactivity. To overcome this limitation, aqueous reactions involving *N*-(3-dihydroxyborylphenyl)succinamic acid, succinimidyl ester should only be undertaken in the presence of at least a 10-fold molar exess of a phenylboronic acid complexing ligand. Compounds found to be useful in this regard include mannitol and catechol. Phenylboronate complexes temporarily prepared for this purpose may be readily dissociated upon neutralization of the solution. Primary amine containing buffers including Tris and glycine must be avioded, due to their potential reactivity. Solid-phase supports having pendant primary amine moieties, including blotting membranes and microtiter plates, may be functionalized by reaction with phenylboronic acid reagents of Formula XIV to afford solid-phase supports having pendant phenylboronic acid moieties.

### Example III: Preparation of A Thiol Reactive Reagent of General Formula XIV (3-Maleimidophenyl)boronic Acid

Ethyl acetate (400 ml) is cooled in an ice bath to approximately 0° C. Maleimide (7.76 grams,) is added to the cooled solvent with strring, followed by *N*-ethylmorpholine (10.19 ml). Methyl chloroformate (6.26 ml,) is added dropwise from an addition funnel at an appropriate rate so as to maintain the temperature of the reaction below 3° C. After completion of the addition, the reaction is stirred for an additional 30 min. while maintaining the temperature below 3° C. The resulting mixture is filtered through a Buchner funnel, and the precipitate washed with a small volume of ethyl acetate. The filtrate and wash are combined, and then washed with ice cold water (100 ml). The organic phase is dried over anhydrous Na₂SO₄, and then concentrated on a rotary evaporator. The product is dissolved in a mixture of ethyl acetate and isopropyl ether (40:60 v/v, 75 ml) in a water bath at 60° C, and then allowed to recrystallize at room temperature. Crystals of N-methoxycarbonylmaleimide are washed with isopropyl ether (2 X 20 ml), and then dried overnight *in vacuo.*
(3-Aminophenyl)boronic acid (1.26 grams, 0.01 mole) is dissolved in saturated NaHCO₃ (50 ml) by briefly heating the mixture on a hot plate. The solution is cooled in an ice bath to approximately 0° C, and *N*-methoxycarbonylmaleimide (1.55 grams, 0.01 mole) added with vigorous stirring. After 10 min. the solution is diluted with water (200 ml) and then stirred at room temperature for 30 to 40 min. The pH is adjusted to approximately 5.5 by addition of 1M H₂SO₄, and the precipitate collected by filtration. The precipitate is washed with 1M H₂SO₄ (2 X 50 ml), and then dried overnight *in vacuo* over NaOH pellets to afford 1.39 grams (64% yield) of (3-maleimidophenyl)boronic acid. The structure was confirmed by 300 MHz, ¹H NMR spectrometry in d₆ DMSO.

### Example IV: Applications of A Thiol Reactive Reagent of General Formula XIV

Proteins containing disulfide bonds (cystine residues) or cysteine residues may be modified with thiol reactive phenylboronic acid reagents of Formula XIV. Disulfide bonds are first reduced, if required, by reaction with 2-mercaptoethanol or dithiothreitol, in an alkaline aqueous buffer. The excess reducing reagent is removed by dialysis or desalting, and the protein reacted with (3-maleimidophenyl)boronic acid in 25 to 100 mM phosphate buffer, pH 7.0 to 7.5, for 1 hour at 4∞C, to afford a semiconjugate having pendant phenylboronic acid moieties covalently affixed to the protein. Proteins which lack thiol moieties may be functionalized by reaction with a thiolating reagent, and then modified as described above. Thiolating reagents which have proven useful for this purpose include *N*-hydroxysuccinimidyl 3-(2- pyridyldithio)propionate, *N*-hydroxysuccinimidyl S-acetylthioacetate and 2-iminothiolane.

### Example V: Preparation of An Aldehyde Reactive Reagent of General Formula XIV N-(3-Dihydroxyborylphenyl)succinamic Acid Hydrazide

Methanol (10 ml) is cooled in an ice bath to approximately 0° C, and thionyl chloride (1 ml) slowly added. To the resulting stirred solution is added *N*-(3-dihydroxyborylphenyl)-succinamic acid (1.25 grams, 0.005 mole), prepared as described in Example I, and the reaction is stirred overnight at room temperature. The solution is concentrated on a rotary evaporator to afford a white crystalline material which is coevaporated twice from methanol (2 X 10ml) to remove residual thionyl chloride. The product is dissolved in methanol (5 ml) and hydrazine hydrate (1 ml) added. The resulting solution is stirred overnight at room temperature. A precipitate forms within a few hours. The precipitate is collected by filtration, washed with cold methanol and dried overnight *in vacuo* over NaOH pellets to afford 1.11 grams (88% yield) of *N*-(3-dihydroxyborylphenyl)succinamic acid hydrazide. The structure was confirmed by 300 MHz, ¹H NMR spectrometry in d₆ DMSO.

### Example VI: Application of An Aldehyde Reactive Reagent of General Formula XIV

Glycoproteins, and particularly antibodies, may be conjugated with an aldehyde reactive phenylboronic acid hydrazide reagent after treatment of the protein with from 5 to 20 mM sodium meta periodate (NaIO₄), in from 0.1 to 0.5 M sodium acetate buffer at pH 5 to 6, containing up to 0.2 M sodium chloride, at 0° C, for from 30 minutes to 4 hours. The excess periodate is removed by desalting, and the activated protein, having pendant adjacent aldehyde moieties resulting from periodate oxidation of carbohydrate residues having 1,2-diol moieties, is condensed with the hydrazide reagent, for from 1 to 24 hours at room temperature, to afford a semiconjugate having pendant phenylboronic acid moities covalently appended to the protein through a Schiff base (an imine) type linkage. The stability of the linkage to the protein may be increased, if desired, by mild sodium cyanoborohydride reduction of the Schiff base to the corresponding alkylamine. It is important to note that periodate oxidation of a glycoprotein activates the protein toward reaction with a hydrazide type reagent while simultaneously removing most naturally occuring phenylboronic acid complexing moities (coaxial 1,2-diols) associated with glycoproteins.

### Example VI: Synthesis of Phenylboronic Acid Complexing Reagents of General Formula XI

Reagents of General Formula XI, wherein group W* is selected from either NH₂, NHCH₃ and NHOH, and wherein group Q is O, are derived from either 4-aminosalicylic acid or 5- aminosalicylic acid. 4- or 5-aminosalicylic acid is first esterified to afford either methyl 4-aminosalicylate or methyl 5-aminosalicylate, respectively. The ester is neutralized and then amidated by reaction with an amine selected from either ammonia, methylamine and hydroxylamine, to afford the 4- or 5-aminosalicylamide of formula XXII: 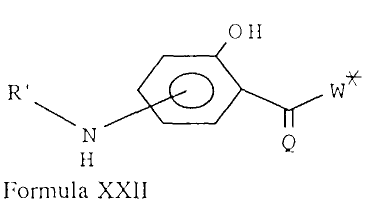 wherein R' is H, W* is selected from NH₂, NHCH₃ and NHOH and Q is O. This compound is next condensed with an activated carboxylic acid, preferably selected from, but not limited to, succinic anhydride, methyl succinyl chloride, maleic anhydride, *N*-methoxycarbonylmaleimide, 3-bromopropionyl chloride, 3-iodopropionyl chloride, iodoacetyl chloride, bromoacetyl chloride and chloroacetyl chloride, to afford the corresponding 4- or 5- amidosalicylamide wherein Q is O; W/* is NH₂, NHCH₃ or NHOH; and R becomes an amide of formula Z"-CO- where Z" is CH₂CH₂COOH, CH₂CH₂COOCH₃, CH=CHCOOH, CH₂CH₂Br, CH₂CH₂I, CH₂I, CH₂Br and CH₂Cl.

Such compounds wherein Z" is selected from either CH₂l and CH₂Br are useful as thiol reactive reagents suitable for appending a phenylboronic acid complexing moiety to *a bioactive species* having pendant thiol groups. Such compounds wherein Z" is CH=CHCOOH may be further functionalized by ring closure to afford a thiol reactive maleimide reagent suitable for appending a phenylboroic acid complexing moiety to a *bioactive species* having pendant thiol groups. When Z" is CH₂CH₂COOCH₃, the compound may be further functionalized by reaction with hydrazine hydrate to afford a hydrazide reagent wherein Z" is CH₂CH₂CONHNH₂, suitable for appending a phenylboronic acid complexing moiety to a *bioactive species* having pendant aldehyde groups (which result from periodate oxidation of carbohydrate residues). When Z" is selected from either CH₂CH₂Br and CH₂CH₂I it may be further functionalized by reaction with potassium thiolacetate to afford an intermediate which, upon deprotection, affords the thiol containing compound wherein Z" is CH₂CH₂SH. The thiol containing compound may be activated by reaction with a reagent preferably selected from, but not limited to 2,2'-dithiodipyridine, 4,4'-dithiodipyridine and 2,2'-dithiodi(3-nitropyridine), to prepare an activated disulfide containing reagent suitable for appending a phenylboronic acid complexing moiety to a *bioactive species* having pendant thiol groups, through a cleavable disulfide bond.

When W* in formula XXII is selected from either NH₂ and NHCH₃, and group Z" is CH₂CH₂COOH, further functionalization is possible by reaction with dicyclohexylcarbodiimide (DCC) and a reagent preferably selected from, but not limited to, either N-hydroxysuccinimide (NHS) and *N*-hydroxysulfosuccinimide (SNHS), to afford an activated ester reagent suitable for appending a phenylboronic acid complexing moiety to a *bioactive species* having pendant amine groups. Activated esters of formula XXII, wherein W* is selected from either NH₂ and NHCH₃, and wherein Z" is CH₂CH₂CO-NHS, are useful synthetic intermediates for the preparation of reagents of Formula XI, wherein group Z is comprised of an alkyl chain or polyethyleneglycol chain of at least 6 carbon equivalents in length.

When W* is NHOH such compounds cannot be routinely employed to prepare reagents having activated ester moieties, due to problems which arise when the carboxylic acid group is activated in the presence of both NHS and the benzohydroxamic acid group associated with the phenylboronic acid complexing moiety. This represents a limitation, due to the popularity of N-hydroxysuccinimide reagents, and the fact that only reagents of formula XI, wherein group W* is selected from either NHOH and NHOCH₃, form stable complexes of over a broad pH range. To overcome this limitation, an alternate synthetic route is employed to prepare activated ester reagents of formula XI, wherein group W* is NHOCH₃, and Q is O. 4-Aminosalicylic acid or 5-aminosalicylic acid is condensed with methyl succinyl chloride to afford a compound of formula XXII, wherein group W* is OH, and wherein group Z" is CH₂CH₂COOCH₃. Subsequent reaction with N,N-carbonyldiimidazole (CDI) followed by addition of methoxylamine hydrochloride, affords a compound wherein W* is NHOCH₃ and Z' is CH₂CH²COOCH₃. Alkaline hydrolysis of the carboxylic acid ester group affords a compound having a free carboxylic acid group. Subsequent activation of the carboxylic acid group by reaction with DCC and a reagent preferably selected from, but not limited to, either NHS and SNHS, affords an activated ester reagent wherein W* is NHOCH₃ and Z" is succinimidoxy, suitable for appending a phenylboronic acid complexing moiety to a bioactive species having pendant amine groups. Such *N*-hydroxysuccinimidyl esters are useful synthetic intermediates for the preparation of reagents of Formula XI, wherein group Z is comprised of an alkyl chain or polyethyleneglycol chain, of at least 6 carbon equivalents in length.

The *N*-Hydroxysuccinimidyl esters may be further functionalized by reaction with a reagent preferably selected from, but not limited to 6-aminohexanoic acid, 4-aminobutanoic acid, *N-tert*-butoxycarbonyl-1,6-diaminohexane (*N*-Boc-1,6-diaminohexane) and *N*-Boc-1,4-diaminobutane, to afford, after removal of the Boc protecting group, if required, a compound having an extended spacer and either a terminal carboxylic acid group or a terminal amine group. The aforementioned reagents, having pendant carboxylic acid groups, are useful for the preparation NHS ester, SNHS ester and hydrazide containing reagents having long spacers, which are useful to overcome steric hindrance know to be associated with biological macromolecules of high molecular weight. Similarly, the aforementioned reagents, having pendant amine groups, are useful for the preparation iodoacetamide, maleimide and activated disulfide containing compounds having long spacers. Additionally, the aforementioned reagents having either pendant carboxylic acid groups or pendant amine groups groups are useful synthetic intermediates for the preparation of solid-phase supports.

### Example VII: Synthesis of Phenylboronic Acid Complexing Reagents of Formula XII

Reagents of Formula XII, wherein group W* is selected from either NH₂, NHCH₃ and NHOH, and wherein group Q is O, are derived from either 4- or 5-aminosalicylic acid in a manner analogous to that employed to prepare reagents of Formula XI. The 4-or 5- amino salicylamide prepared as previously described is condensed with an activated dicarboxylic acid preferably selected from, but not limited to, succinyl chloride, adipoyl chloride, adiptic acid diisobutylcarbonate, suberoyl chloride, 3,3'-dithiopropionyl chloride, 3,6,9-trioxaundecanedioyl chloride and 3,6,9-trioxaundecanedioic acid diisobutylcarbonate, to afford a compound of formula XII, wherein group W* is selected from either NH₂, NHCH₃ and NHOH, wherein group Q is O, and wherein group Z* is selected from, but not limited to, either (CH₂)₂, (CH₂)₄, (CH₂)₆, (CH₂)₂SS(CH₂)₂ and CH₂(OCH₂CH₂)₂OCH₂, respectively.

Reagents of formula XII, wherein group W* is NHOCH₃, and wherein group Q is O, are prepared by an alternate synthetic route, in a manner analogous to the preparation of reagents of formula XI, wherein group W* is NHOCH₃. Either 4- or 5-aminosalicylic acid is condensed with an activated dicarboxylic acid preferably selected from, but not limited to, succinyl chloride, adipoyl chloride, adiptic acid diisobutylcarbonate, suberoyl chloride, 3,3'-dithiopropionyl chloride and 3,6,9-trioxaundecanedioyl chloride and 3,6,9-trioxaundecanedioic acid diisobutylcarbonate, to afford a compound of formula XII, wherein group W* is OH, wherein group Q is O, and wherein group Z* is selected from, but not limited to, either (CH₂)₂, (CH₂)₄, (CH₂)₆, (CH₂)₂SS(CH₂)₂ and CH₂(OCH₂CH₂)₂OCH₂, respectively.

Subsequent reaction of a compound of formula XII, wherein W* is OH, wherein Q is O, and wherein Z* is selected from, but not limited to, either (CH₂)₂, (CH₂)₄, (CH₂)₆, (CH₂)₂SS(CH₂)₂ and CH₂(OCH₂CH₂)₂OCH₂, with CDI followed by addition of methoxylamine hydrochloride, affords a compound wherein group W* is NHOCH₃, and wherein groups Q and Z* are as were previously defined.

### Example VIII: Preparation of 4-Amino-2-hydroxybenzohydroxamic Acid

Absolute methanol (100 mL) and fresh concentrated H₂SO₄ are carefully combined in a 250 mL round button flask with continuous stirring (exothermic). 4-Aminosalicylic acid (10.0 grams, 65.4 mmol) is added to produce a dark solution, which is heated under reflux for 6 hours. The product is allowed to cool and then concentrated on a rotary evaporator to approximately half the original volume. At this point a solid precipitate appears. The concentrate is poured into 400 mL of water, and the resulting suspension titrated to approximately pH 3, by stepwise addition of 5N NaOH (to pH 6.5), followed by solid Na₂CO₃, with evolution of CO₂ gas. The concentrate was chilled on ice, and the resulting precipitate collected by filtration. The filtrate is washed with cold water, and then dried *in vacuo* over NaOH pellets, to afford 9.6 grams (88% yield) of methyl 4-aminosalicylate, a pale lavendar powder (m.p. 115-117° C). The structure was confirmed by ¹H NMR spectrometry in d₆-acetone.

NaOH (4.0 grams) in 16 mL of water is carefully added to hydroxylamine hydrochloride (2.8 grams, 40 mmol) and 20 grams of ice. After dissolution, Na₂SO₃ (0.4 grams) is added, followed by methyl 4-aminosalicylate (3.35 grams, 20 mmol). The resulting solution is stirred for up to 24 hours at room temperature, the course of the reaction is monitored every few hours by reverse phase HPLC. The resulting solution is chilled on ice and acidified by addition of 25% H₂SO₄. A precipitate is first formed at approx. pH 6. The pH is finally adjusted to approx. pH 4, and the pale tan precipitate collected by filtration. The product is dried *in vacuo* over P₂O₅, to afford 3.0 grams (89% yield) of 4-amino-2-hydroxybenzohydroxamic acid (m.p. 180-181° C). The structure was confirmed by ¹H NMR spectrometry in d₆-DMSO.

4-Amino-2-hydroxybenzohydroxamic acid is a key synthetic intermediate in the preparation of reagents of both General Formulas I and II, wherein group X is NHOH, and wherein group Y is O. Reagents having the 2-hydroxybenzohydroxamic acid moiety can form bioconjugates which are stable in buffered aqueous solutions over the approximate pH range 2.5 to 11.5.

### Example IX: Preparation of An Aldehyde Reactive Phenylboronic Acid Complexing Reagent of Formula XI

To an ice-cooled, stirred solution of 4-Amino-2-hydroxybenzohydroxamic acid (8.4 grams, 0.05 mol), prepared as described above, in 150 mL of water containing NaHCO₃ (7.0 grams, 0.02 mol), 3-carbomethoxypropionyl chloride (9.0 grams, 0.06 mol) is added dropwise over 15 min. After stirring at 0-5° C for 1 hr., the solution is acidified with cold 6N HCI. The precipitate is collected and dried *in vacuo* over NaOH pellets, to afford 13.5 grams (96% yield) of crude *N*-4- (3-carbomethoxypropionamido)-2-hydroxy-benzohydroxamic acid, which is utilized without further purification.

To a solution of *N*-4-(3-carbomethoxypropionamido)-2-hydroxybenzohydroxamic acid (10 grams, 0.035 mol) in 50 mL of methanol is added hydrazine hydrate (12 mL). The reaction is allowed to proceed overnight at room temperature, and the product is filtered and washed with ether. The product is recrystallized twice from dimethylformamide to afford 7.3 grams (78% yield) of *N*-4-(3-hydrazidopropionamido)-2-hydroxybenzohydroxamic acid.

### Example X: Application of An Aldehyde Reactive Phenylboronic Acid Complexing Reagent

Glycoproteins, and particularly antibodies, may be conjugated with an aldehyde reactive phenylboronic acid complexing hydrazide reagent after treatment of the protein with from 5 to 20 mM sodium meta periodate (NaIO₄), in from 0.1 to 0.5 M sodium acetate buffer at pH 5 to 6, containing up to 0.2 M sodium chloride, at 0° C, for from 30 minutes to 4 hours. The excess periodate is removed by dialysis or desalting, and the activated protein, having pendant adjacent aldehyde moieties resulting from periodate oxidation of carbohydrate residues having adjacent coaxial 1,2-diol moities, is condensed with the hydrazide reagent, for from 1 to 24 hours at room temperature, to afford a semiconjugate having pendant phenylboronic acid complexing moities covalently appended to the protein throught a Shiff base (an imine) type linkage.
The stability of the linkage to the protein may be increased, if desired, by mild sodium cyanoborohydride reduction of the Schiff base to the corresponding alkylamine. It is important to note that sodium meta periodate oxidation of a glycoprotein activates the protein toward reaction with a hydrazide type reagent while simultaneously removing most naturally occuring phenylboronic acid complexing sites (coaxial 1,2-diols) associated with glycoproteins.

### Example XI: Preparation of A Thiol Reactive Phenylboronic Acid Complexing Reagent of Formula XI

To an ice-cooled, stirred solution of 5-Aminosalicylamide (10.0 grams, 0.073 mol), in 300 mL of water containing NaHCO₃ (42.0 grams, 0.5 mol), iodoacetyl chloride (18.4 grams, 0.09 mol) is added dropwise over 15 min. After stirring at 0-5° C for 1 hr., the solution is acidified with cold 6N HC1. The precipitate is collected and dried *in vacuo* over NaOH pellets, to afford 21.3 grams (96% yield) of crude 5-(iodoacetamido)salicylamide.

### Example XII: Application of A Thiol Reactive Phenylboronic Acid Complexing Reagent

Proteins containing disulfide bonds (cystine residues) or cysteine residues may be modified with a thiol reactive phenylboronic acid complexing reagent, such as 5-(iodoacetamido)-salicylamide. Disulfide bonds are first reduced, if required, by reaction with 2-mercaptoethanol or dithiothreitol, in alkaline aqueous solution which has been throughly degassed. The excess reducing reagent is removed by dialysis or desalting, and the protein reacted with the alkylating reagent in neutral aqueous solution, for 1 hour at 4° C, to afford a semiconjugate having pendant phenylboronic acid complexing moities covalently appended to the protein. Upon completion of the reaction, the excess reagent is removed by desalting.

### Example XIII: Preparation of An Amine Reactive Phenylboronic Acid Complexing Reagent of Formula XI

To an ice-cooled, stirred solution of 4-Aminosalicylic acid (7.7 grams, 0.05 mol), prepared as described above, in 150 mL of water containing NaHCO₃ (7.0 grams, 0.02 mol), 3- carbo-methoxypropionyl chloride (9.0 grams, 0.06 mol) is added dropwise over 15 min. After stirring at 0-5° C for 1 hr., the solution is acidified with cold 6N HCl. The precipitate is collected and dried *in vacuo* over NaOH pellets, to afford 11.9 grams (89% yield) of crude *N*-4-(3-carbomethoxy-propionamido)salicylic acid, which is utilized without further purification.

To a vigorously stirred solution of *N*-4-(3-carbomethoxypropionamido)salicylic acid (10.0 grams, 0.036 mol) in 50 mL of tetrahydrofuran is added stepwise 1,1'-carbonyldiimidazole (5.84 grams, 0.036 mol) and methoxylamine hydrochloride (3.0 grams, 0.036 mol). The vessel is fitted with a drying tube and the reaction vigorously stirred for 30 min. at room temperature. Imidazolium hydrochloride, which separated during the course of the reaction, is removed by filtration. The filtrate is concentrated on a rotary evaporator to afford an amber oil, which is dissolved in 10 mL of warm tetrahydrofuran, and then added to 150 mL of 2N H₂SO₄. The precipitate is collected by filtration, washed with 2N H₂SO₄, washed with water, and then dried overnight *in vacuo* over NaOH pellets to afford 10.0 grams (94% yield) of *N*-4-(3-carbomethoxy-propionamido)-2-hydroxy-*O*-methylbenzohydrox amic acid.
*N*-4-(3-Carbomethoxypropionamido)-2-hydroxy-*O*-methylbenzohydro xamic acid (7.4 grams, 0.025 mol) is dissolved in 25 mL of 0.2N methanolic LiOH. The solution is stirred overnight at room temperature under nitrogen. Methanol is removed on a rotary evaporator, and the residue dissolved 150 mL of water. Acidifation of the solution to approximately pH 2 with 2N H₂SO₄ is followed by extraction into 100 mL of ether. A second extration into ether is followed by drying of the combined ether extracts over anhydrous Na₂SO₄. The product is concentrated-on a rotary evaporator and then dried overnight *in vacuo* over P₂O₅, to afford 6.28 grams (89% yield) of *N*-4- succinamido-2-hydroxy*-O*-methylbenzohydroxamic acid.

*N*-4-succinamido-2-hydroxy-*O*-methylbenzohydroxamic acid (5.65 grams, 0.02 mol) is dissolved in 50 mL of hot dimethylformamide and allowed to cool to room temperature. To the stirred solution is added *N*-hydroxysuccinimide (2.3 grams, 0.02 mol) followed by a freshly prepared solution of dicyclohexylcarbodiimide (4.1 grams, 0.02 mol) in 10 mL of dimethyl-formamide. The resulting suspension is stirred overnight at room temperature. Dicyclohexylurea is filtered from solution, and the filtrate concentrated on a rotary evaporator to a minimum volume. The residue is precipitated with ether, and the precipitate collected by filtration, washed with ether, washed with 2-propanol, and then dried briefly *in vacuo* over P₂O₅, to afford 5.6 grams (74% yield) of *N*-4-succinamido-2-hydroxy*-O*-methlybenzohydroxamic acid succinimidyl ester. The product is storred in a freezer at -15°C.

### Example XIV: Application of An Amine Reactive Phenylboronic Acid Complexing Reagent

Proteins may be conjugated with amine reactive phenylboronic acid complexing reagents by reaction with the side-chain e-amino groups of lysine residues, to afford a semiconjugate having pendant phenylboronic acid complexing moities covalently appended to the protein through stable amide bonds. Mildly alkaline aqueous buffers, in the pH range 7.8 to 8.8, should be employed so as to insure that the amino group is unprotonated, while minimizing hydrolysis of the NHS ester. Primary amine containing buffers including Tris and glycine must be avoided, so as to avoid cross-reactivity. Solid-phase supports having pendant primary amine moities may be functionalized, in an analogous manner, by reaction with phenylboronic acid complexing reagents to afford solid-phase supports having pendant phenylboronic acid complexing moities.

### Example XV: Preparation of A Phenylboronic Acid Complexing Reagent of Formula XII

4-Amino-2-hydroxybenzohydroxamic acid (6.4 grams, 0.038 mol) is dissolved in 50 mL of dry dichloromethane. Triethylamine (5.3 mL, 0.038 mol) is added followed by the dropwise addition of a solution of 3,6,9-trioxaundecanedioyl chloride (5.0 grams, 0.019 mol) in 50 mL of dry dichloromethane, over a period of 2 hours.
Triethylammonium hydrochloride is removed by filtration, and the filtrate washed with water (2 X 100 mL), saturated NaHCO₃ (2 X 100 mL), saturated NaCI (100 mL), and dried over anhydrous Na₂SO₄. The solvent is removed on a rotary evaporator and the residue recrystallized from methanol (100 mL) to afford 6.0 grams (61% yield) of colorless crystals. The structure was confirmed by ¹H NMR spectrometry in d₆DMSO.

### Example XVI: Application of A Phenylboronic Acid Complexing Reagent of Formula XII

Proteins which have been conjugated to phenylboronic acid may be cross-linked by the action of reagents of Formula XII. This process is particularly useful for the preparation of protein aggregates, which are useful for modifying the properties of protein stability and solubility. Additionally, enzyme aggregates, prepared by cross-linking, are useful for amplifying the sensitivity of ELISA. This principle is exploited in the Avidin-Biotin system by the use of Avidin-Biotin complexes (called ABC complexes), in which a biotinylated enzyme is first employed to form a high molecular weight complex with Avidin (by cross-linking), prior to introduction of the complex into an ELISA for the high sensitivity detection of a biotinylated antibody.

### Example XVII: General Synthesis of Phenylboronic Acid Complexing Reagents of Formula XVIII

Reagents of formula XVIII, e.g., wherein X is selected from either H, CH₃ and C₆H₅, and wherein Y is O, are prepared by condensation of N-hydroxyphthalimide with a compound of the general formula R₁-Z-R₂, wherein R₁ is selected from either Br, Cl and I, and is preferably Br, and wherein R₂ is selected from Br, Cl, I, CO₂H and CO₂CH₃, and is preferably selected from Br, CO₂H and CO₂CH₃, and wherein Z is as defined for XVIII, e.g., preferably a spacer which is selected from either an alkyl chain or polyether chain, of from 2 to 12 carbon equivalents in length, and which may contain intermediate amide functionalities, and is preferably (CH₂)ₙ, wherein n = 2 to 6. or (CH₂CH₂O)ₙ, wherein n = 2 to 4.
(a) In the initial reaction, a compound of the general formula R₁-Z-R₂ is heated in dimethylforamide with one equivalent of N-hydroxyphthalimide at from 40° to 100° C until solution is obtained. The solution is then allowed to cool to room temperature at one equivalent of triethylamine added, producing a dark red color associated with the *N*-hydroxyphthalimide anion. The solution is stirred at room temperature for from one to four days, the progress of the reaction being monitored by thin-layer chromatography (TLC). Upon completion of the reaction, water is added to effect precipitation of the product, which is washed with water and dried at room temperature, to afford a product where R₁ is phthalimido and R₂ are as were previously defined. The product wherein R₂ is selected from either Br, Cl and I, is condensed with a reagent preferably selected from, but not limited to, C₆H₄(CO)₂NK, CH₃COONa and CH₃COSK. Conditions vary depending upon the choice of desired product, but generally involve the addition of 1.1 equivalents of either C₆H₄(CO)₂NK, CH₃COONa or CH₃COSK by refluxing in a polar solvent selected from either acetic acid, dimethylformamide, methanol or ethanol, for from 1 to 24 hours.
(b) The product of (a) is then subjected to acid catalyzed hydrolysis of the phthalimide group such that R₁ becomes NH₂ and R₂ is acylated to R₂Ac, wherein R₂Ac is selected from either N(CO)₂C₆H₄, OCOCH₃ and SCOCH₃, and wherein Z is as was previously defined. Acid catalyzed hydrolysis of the phthalimide group when R₂ is CO₂CH₃, affords a product wherein R₂ is selected from either CO₂H and CO₂CH₃, and wherein Z is as was previously defined. The phthalimide group is by refluxing briefly for from 15 to 60 min in either concentrated hydrochloric acid, concentrated hydrochloric acid in acetic acid, 30% hydrobromic acid, or 48% hydrobromic acid. In each instance, the phthalic acid by-product is filtered from the resulting solution, after allowing to cool to room temperature. The volume is reduced and the product neutralized with either NaOH, NaHCO₃ or Na₂CO₃. Extraction into either ether or ethyl acetate and subsequent concentration *in vacuo* affords the product.
(c) The product of (b) is then condensed with a reagent selected from either salicylaldehyde, 2-hydroxyacetophenone and 2-hydroxydiphenylketone, to afford the corresponding carbonimidoyl products at R₁. Condensation of products with a reagent selected from either salicylaldehyde, 2-hydroxyacetophenone and 2-hydroxy-diphenylketone is achieved by refluxing in either methanol or 90% ethanol, at 60°C, for from 4 to 12 hours, the progress of the reaction being monitored by TLC. The product is concentrated *in vacuo*, then dried in a dessicator overnight.
(d) Products of (c) are deprotected by base catalyzed hydrolysis in warm aqueous K₂CO₃ or NaOH, for from 8 to 24 hours, to afford products of general formula P5, wherein R₃ and Q are as were previously defined.. The product is acidified with HCl, extracted into ethyl acetate, dried over anhydrous MgSO₄, and concentrated *in vacuo.* The protecting group is removed by reaction with hydrazine hydrate in refluxing ethanol, for from 12 to 48 hours. The precipitated phthalhydrazide is filtered from solution, the solution concentrated, the product extracted into ethyl acetate, dried over anhydrous MgSO₄, and concentrated *in vacuo.*
(e) The final product is prepared by activation of the amino, hydroxyl, thiol, or carboxylic acid groups associated with the products. Amino groups may be activated by reaction with a reagent preferably selected from, but not limited to, bromoacetic anhydride, iodoacetic anhydride and maleic anhydride. Hydroxy groups may be activated by reaction with a reagent preferably selected from, but not limited to, 2,2,2-trifluroethaneulfonyl chloride, pentafluorobenzenesulfonyl chloride, toluenesulfonyl chloride and 2-cyanoethyl-*N,N*-diisopropylchlorophosphoramidite. Thiol groups may be activated by reaction with a reagent preferably selected from, but not limited to, 2-thiopyridone, 4-thiopyridone, and 3-nitro-2-mercaptopyridine. Carboxylic acid groups may be activated by reaction with a reagent preferably selected from either dicyclohexylcarbodiimide and 1-ethyl-3-(3-dimethylaminopropyl)carbodiimide, in the presence of a reagent preferably selected from, but not limited to, *N*-hydroxysuccinimide and *N*-hydroxysulfosuccinimide. Alternatively, carboxylic acid groups may be esterified with an alcohol preferably selected from either methanol and ethanol and then further functionalized by reaction with a reagent preferably selected from either hyrazine hydrate and hydroxylamine.

Products of general formula XVIII, wherein X is selected from either H, CH₃ and C₆H₅, and wherein Y is CH₂, are prepared as previously outlined by substituting potassium phthalimide for *N*-hydroxyphthalimide in the initial step of the synthesis.

### Example XVIII: General Synthesis of Phenylboronic Acid Complexing Reagents of Formula XIX

(a) Reagents of general formula XIX, wherein Y is O, are prepared in a manner analogous to that described above, the synthesis proceeding through the steps (a) and (b) exactly as described in the preceding example. The product of step (b) of the preceding example is then condensed with a reagent preferably selected from, but not limited to, either 2-acetoxybenzoyl chloride and 2-benzyloxy-benzoyl chloride, to afford the corresponding amide. Condensation of the products with a reagent preferably selected from, but not limited to, either 2-acetoxybenzoyl chloride and 2-benzyloxybenzoyl chloride, is achieved by stirring in dichloromethane containing one equivalent of triethylamine, for 1 hour at room temperature, the progress of the reaction being monitored by TLC. Triethylammonium hydrochloride is filtered from the solution. The filtrate is washed with water, dried over anhydrous MgSO₄, and concentrated *in vacuo.*
(b) The products of the preceding step are deprotected (e.g. deacylated at R₂) by base catalyzed hydrolysis in warm aqueous K₂CO₃ or NaOH, for from 8 to 24 hours. The product is acidified with HC1, extracted into ethyl acetate, dried over anhydrous MgSO₄, and concentrated *in vacuo.* If an acetoxy protecting group had been employed to protect the phenolic hydroxyl group during the preparation of the products, it would also be removed at this time, precluding the necessity for the synthetic step which follows. The protecting group is removed by reaction with hydrazine hydrate (N₂H₂∘XH₂O) in refluxing ethanol, for from 12 to 48 hours. The precipitated phthalhydrazide is filtered from solution, the solution concentrated, the product extracted into ethyl acetate, dried over anhydrous MgSO₄, and concentrated *in vacuo.*
(c) The products of step (b) are further deprotected, if required, by removal of the benzyloxy protecting group by catalytic hydrogenation. Catalytic hydrogenation proceeds over paladium-charcoal catalyst in anhydrous absolute ethanol, for from 2 to 12 hours. The catalyst is removed by filtration and the product concentrated *in vacuo.*
(d) The final product is prepared by activation of the amino, hydroxyl, thiol, or carboxylic acid groups associated with the products. Amino groups may be activated by reaction with a reagent preferably selected from, but not limited to, bromoacetic anhydride, iodoacetic anhydride and maleic anhydride. Hydroxy groups may be activated by reaction with a reagent preferably selected from, but not limited to, 2,2,2-trifluroethaneulfonyl chloride, pentafluorobenzenesulfonyl chloride, toluenesulfonyl chloride, and 2-cyanoethyl-*N,N*-diisopropylchlorophosphoramidite. Thiol groups may be activated by reaction with a reagent preferably selected from, but not limited to, 2-thiopyridone, 4-thiopyridone, and 3-nitro-2-mercaptopyridine. Carboxylic acid groups may be activated by reaction with a reagent preferably selected from either dicyclohexylcarbodiimide and 1-ethyl-3-(3-dimethylaminopropyl)carbodiimide, in the presence of a reagent preferably selected from, but not limited to, *N*-hydroxysuccinimide and *N*-hydroxysulfosuccinimide. Alternatively, carboxylic acid groups may be esterified with an alcohol preferably selected from either methanol and ethanol and then further functionalized by reaction with a reagent preferably selected from either hyrazine hydrate and hydroxylamine. If activation of the final product is incompatible with the presence of a phenolic hydroxyl group, then the products may be first activated and the benzyloxy protecting group subsequently removed, provided that the activated form is stable toward catalytic hydrogenation.

Products of general formula XII, wherein group Z is CH₂ are prepared as previously outlined by substituting potassium phthalimide for *N*-hydroxyphthalimide in the initial step of the synthesis.

### Example XIX: Preparation of An Aldehyde Reactive Phenylboronic Acid Complexing Reagent of General Formula XVIII

In the initial step of the synthesis methyl 6-bromohexanoate is condensed with *N*-hydroxyphthalimide by stirring in dimethyl-formamide containing one equivalent of triethylamine for 24 hours. The product is precipitated by pouring into water, collected by filtration, washed with water, dried in a vacuum dessicator, and used without further purification.

In the second step of the synthesis the crude product obtained above is refluxed briefly in a mixture of acetic acid and concentrated hydrochloric acid. After cooling, the precipitated phthalic acid is filtered from solution and the filtrate concentrated and then coevaporated repeatedly from small volumes of water to remove traces of acids. Finally, the aminooxy hydrochloride product is neutralized with NaHCO₃, extrated in ethyl acetate, dried over anhydrous MgSO₄, and concentrated *in vacuo.*

In the third step of the synthesis the aminooxy product obtained above is condensed with one equivalent of 2-hydroxybenzaldehyde by refluxing for 6 hours in 90% ethanol, and then concentrated *in vacuo,* to afford the aldoxime.

Finally, the aldoxime product obtained above is treated with excess hydrazine hydrate by stirring overnight in methanol. The precipitated hydrazide aldoxime product is cooled on an ice bath, filtered from solution, redissolved in methanol, and then concentrated *in vacuo.*

### Example XX: Application of An Aldehyde Reactive Phenylboronic Acid Complexing Reagent

Glycoproteins, and particularly monoclonal antibodies, may be conjugated with an aldehyde reactive phenylboronic acid complexing hydrazide reagent after treatment of the protein with sodium meta periodate in an alkaline aqueous solution, for from 1 to 12 hours. The excess periodate is removed by dialysis or desalting, and the activated protein, having pendant adjacent aldehyde moieties resulting from periodate oxidation of carbohydrate residues having adjacent coaxial 1,2-diol moities, is condensed with the hydrazide reagent, for 1 hour at room temperature, to afford a semiconjugate having pendant phenylboronic acid complexing moities covalently appended to the protein throught a Shiff base type linkage. The stability of the linkage to the protein may be increased by NaCNBH₃ reduction of the Schiff base to the corresponding amine. It is important to note that sodium meta periodate oxidation of a glycoprotein activates the protein toward reaction with a hydrazide type reagent while simultaneously removing most naturally occuring phenylboronic acid complexing sites (coaxial 1,2-diols) associated with glycoproteins.

### Example XXI: Preparation of A Thiol Reactive Phenylboronic Acid Complexing Reagent of General Formula XIX

In the initial step of the synthesis 1,2-bis-(2-iodo-ethoxy)ethane is condensed with *N*-hydroxyphthalimide by refluxing in dimethylformamide containing one equivalent of triethylamine for 3 days. The product is precipitated by pouring into water, collected by filtration, washed with water, dried in a vacuum dessicator, and used without further purification.

In the second step of the synthesis the crude product obtained above, in absolute ethanol, is treated with excess potassium thioacetate and the resulting yellow suspension heated at reflux for 1 hour. The mixture is cooled, filtered, and concentrated *in vacuo,* and the slurry partitioned between ethyl acetate and water. The combined ethyl acetate layers were washed with saturated aqueous NaHCO₃ solution, and water, dried over anhydrous MgSO₄, and concentrated *in vacuo.*

In the third step of the synthesis the product obtained above is refluxed briefly in a mixture of acetic acid and concentrated hydrochloric acid. After cooling, the precipitated phthalic acid is filtered from solution and the filtrate concentrated and then coevaporated repeatedly from small volumes of water to remove traces of acids. Finally, the aminooxy hydrochloride product is neutralized with NaHCO₃, extrated in ethyl acetate, dried over anhydrous MgSO₄, and concentrated *in vacuo.*

In the fourth step of the synthesis the aminooxy product obtained above is condensed with one equivalent of 2-acetoxybenzoyl chloride by stirring for 1 hour at room temperature in dichloromethane containing one equivalent of triethylamine, the progress of the reaction being monitored by TLC. Triethylammonium hydrochloride is filtered from the solution, and the filtrate washed with water, dried over anhydrous MgSO₄, and concentrated *in vacuo.*

In the fifth step of the synthesis the 2-acetoxybenzohydroxamic acid product obtained above, in absolute methanol was throughly degassed with nitrogen and treated with one equivalent of anhydrous K₂CO₃, and the resulting yellow suspension was stirred vigorously for 12 hours. The suspension was filtered and concentrated *in vacuo.*

Finally, the mercapto 2-hydroxybenzohydroxamic acid product is treated with a solution of (methoxycarbonyl)sulfenyl chloride in dry, degassed methanol by stirrng at 0∞C for 1 hour, and the methanol removed *in vacuo.* The product is again dissolved in degassed methanol and treated with one equivalent of 3-nitro-2-mercaptopyridine by stirring at room temperature for 12 hours. The mixture is filtered to remove unreacted 3-nitro-2-mercaptopyridine, and the product concentrated *in vacuo.*

### Example XXII: Application of A Thiol Reactive Phenylboronic Acid Complexing Reagent

Proteins containing disulfide bonds may be conjugated with a thiol reactive phenylboronic acid complexing reagent. Disulfide bonds are first reduced, by reaction with 2-mercaptoethanol or dithiothreitol, in alkaline aqueous solution which has been throughly degassed. The excess reducing reagent is removed by dialysis or desalting, and the protein reacted with the thiol reactive reagent in throughly degassed alkaline aqueous solution, under nitrogen, overnight at 4∞C, to afford a semiconjugate having pendant phenylboronic acid complexing moities covalently appended to the protein throught disulfide linkages. Upon completion of the reaction, the excess reagent is removed by desalting or by thiol exchange chromatography. The phenylboronic acid complexing moities may be removed from the semiconjugate by reduction of the disulfide bond as described above. In this manner, bioconjugates involving semiconjugates prepared from thiol reactive phenylboronic acid complexing reagents may be cleaved.

### Example XXIII: Preparation of An Amine Reactive Phenylboronic Acid Complexing Reagent of General Formula XIX

In the initial step of the synthesis 2-[2-(2-chloroethoxy)ethoxy]ethanol is condensed with N-hydroxyphthalimide by refluxing in dimethylformamide containing one equivalent of triethylamine for 2 days. The product is precipitated by pouring into water, collected by filtration, washed with water, dried in a vacuum dessicator, and used without further purification.

In the second step of the synthesis the crude product obtained above is refluxed briefly in a mixture of acetic acid and concentrated hydrochloric acid. After cooling, the precipitated phthalic acid is filtered from solution and the filtrate concentrated and then coevaporated repeatedly from small volumes of water to remove traces of acids. Finally, the aminooxy hydrochloride product is neutralized with NaHCO₃, extrated in ethyl acetate, dried over anhydrous MgSO₄, and concentrated *in vacuo.*

In the third step of the synthesis the hydroxy aminooxy product obtained above is condensed with one equivalent of 2-benzyloxybenzoyl chloride by stirring for 1 hour at room temperature in dichloromethane containing one equivalent of triethylamine, the progress of the reaction being monitored by TLC. Triethylammonium hydrochloride is filtered from the solution, and the filtrate washed with water, dried over anhydrous MgSO₄, and concentrated *in vacuo.*

In the fourth step of the synthesis the hydroxy 2-benzyloxybenzohydroxamic acid product obtained above is condensed with one equivalent of 2,2,2-trifluoroethanesulfonyl chloride by stirring for 1 hour at room temperature in acetonitrile containing one equivalent of triethylamine. Triethylammonium hydrochloride is filtered from the solution, and the filtrate washed with water, dried over anhydrous MgSO₄, and concentrated *in vacuo.*

Finally, the benzyloxy protecting group is removed by catalytic hydrogenation over paladium-charcoal for 8 hours in anhydrous absolute ethanol. The catalyst is removed by filtration and the product concentrated *in vacuo.*

### Example XXIV: Application of An Amine Reactive Phenylboronic Acid Complexing Reagent

Proteins may be conjugated with amine reactive phenylboronic acid complexing reagents by reaction with the side-chain -amino groups of lysine residues, to afford a semiconjugate having pendant phenylboronic acid complexing moities covalently appended to the protein through stable sulfonamide bonds. Alkaline aqueous buffers should be employed so as to insure that the amino group is unprotonated. Primary amine containing buffers including Tris and glycine must be avoided, so as to avoid cross-reactivity. Solid-phase supports having pendant primary amine moities may be functionalized, in an analogous manner, by reaction with phenylboronic acid complexing reagents to afford solid-phase supports having pendant phenylboronic acid complexing moities.

### Example XXV: Preparation of A Synthetic Oligonucleotide Reactive Phenylboronic Acid Complexing Reagent of General Formula XIX

In the initial step of the synthesis 2-[2-(2-chloroethoxy)ethoxy]ethanol is condensed with *N*-hydroxyphthalimide by refluxing in dimethylformamide containing one equivalent of triethylamine for 2 days. The product is precipitated by pouring into water, collected by filtration, washed with water, dried in a vacuum dessicator, and used without further purification.

In the second step of the synthesis the crude product obtained above is refluxed briefly in a mixture of acetic acid and concentrated hydrochloric acid. After cooling, the precipitated phthalic acid is filtered from solution and the filtrate concentrated and then coevaporated repeatedly from small volumes of water to remove traces of acids. Finally, the aminooxy hydrochloride product is neutralized with NaHCO₃, extrated in ethyl acetate, dried over anhydrous MgSO₄, and concentrated *in vacuo.*

In the third step of the synthesis the hydroxy aminooxy product obtained above is condensed with one equivalent of 2-acetoxybenzoyl chloride by stirring for 1 hour at room temperature in dichloromethane containing one equivalent of triethylamine, the progress of the reaction being monitored by TLC. Triethylammonium hydrochloride is filtered from the solution, and the filtrate washed with water, dried over anhydrous MgSO₄, and concentrated *in vacuo.*

In the fourth step of the synthesis the hydroxy 2-acetoxybenzohydroxamic acid product obtained above is condensed with one equivalent of 2-cyanoethyl-*N,N*-diisopropylchlorophosphoramidite by stirring for 1 hour at room temperature in acetonitrile containing one equivalent of triethylamine. Triethylammonium hydrochloride is filtered from the solution, and the filtrate washed with water, dried over anhydrous MgSO₄, and concentrated *in vacuo.*

In the fifth step of the synthesis the 2-cyanoethyl-*N,N*-diisopropylphosphoramidite 2-acetoxybenzohydroxamic acid obtained above is dissolved in acetonitrile and placed in the auxiliary reservoir of an automated olgionucleotide synthesizer. The product is condensed with the free 5'-OH end of an immobilized synthetic oligonucleotide undergoing synthesis by pyridine catalyzed reaction with the 2-cyanoethyl-*N,N*-diisopropylphosphoramidite reagent in acetonitrile. The solid-phase synthesis is terminated in this manner. In the final step of the syntheis, the product is cleaved from the glass solid-phase support by ammonia lysis overnight with concentrated ammonium hydroxide at from 50 to 60∞C. Ammonia lysis removes the product from the solid-phase support, as well as removing all acyl protecting groups including the acetoxy group associated with the 2-acetoxybenzohydroxamic acid functionality. The product is concentrated by removal of ammonia on a speedvac, and then purified by reverse-phase high performance liquid chromatography (HPLC).

### Example XXVI: Application of A Synthetic Oligonucleotide Reactive Phenylboronic Acid Complexing Reagent

Synthetic oligonucleotides may be conjugated with a 2-cyanoethyl-*N,N-*diisopropylphosphoramidite phenylboronic acid complexing reagents, during the final step of an automated solid-phase oligonucleotide synthesis, to afford synthetic oligonucleotides having 5'- pendant phenylboronic acid complexing moities.

## Claims

1. Bioconjugate complexes of formula A:
BAS-L-Bc-L'-(Bc'-L")ₙ-BAS' (A)
wherein BAS and BAS' are bioactive species (which may be the same or different); L, L', and L" are linkers (which may be the same or different); Bc and Bc' are phenylboronic acid complexes (which may be the same or different) of formula D-E or formula E-D wherein D is a phenylboronic acid moiety and E is a phenylboronic acid complexing moiety wherein the phenylboronic acid complexing moiety includes a nitrogen bonded to the boron of the phenylboronic acid complexing moiety, and n is 0 or 1.

2. Bioconjugate complexes as claimed in claim 1 selected from formulae I through X: 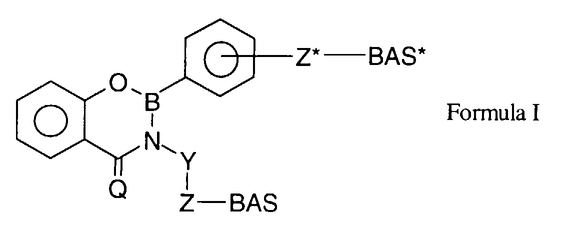 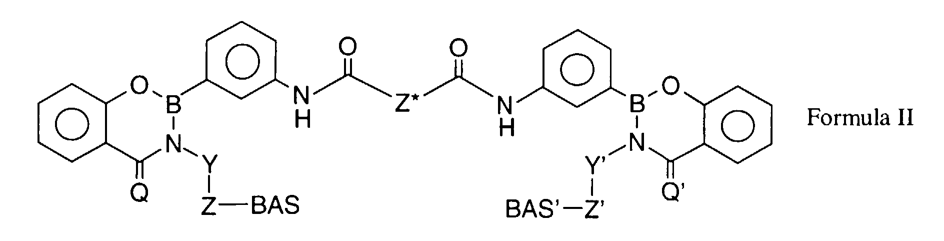 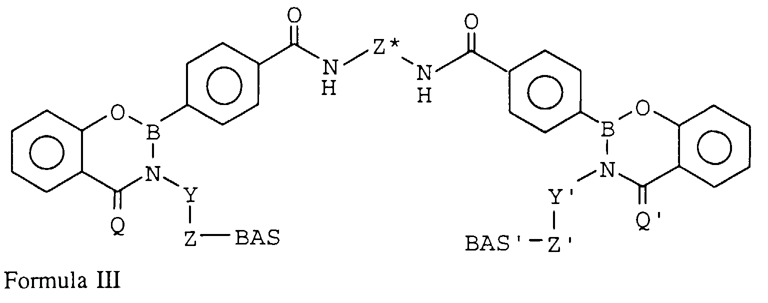 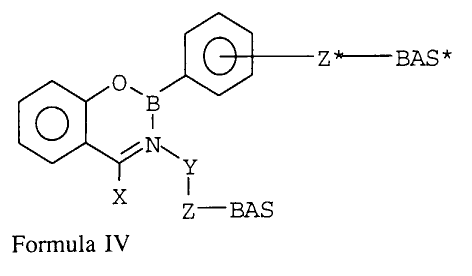 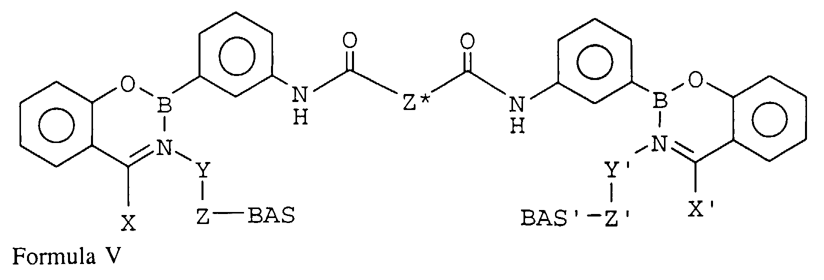 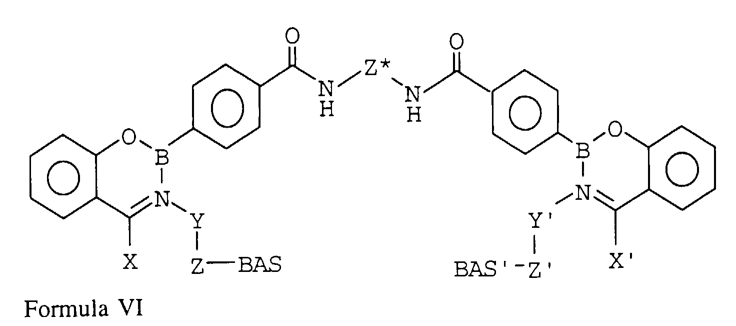 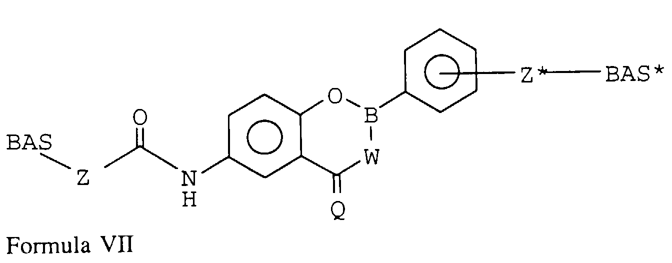 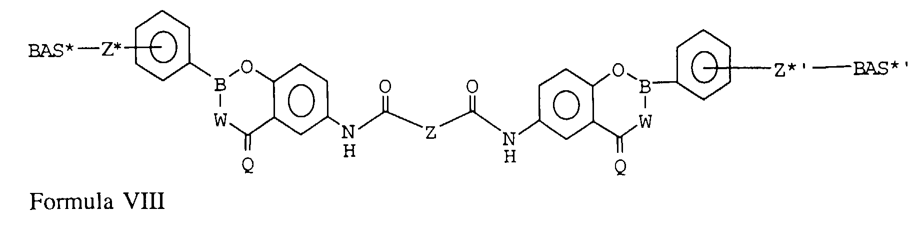 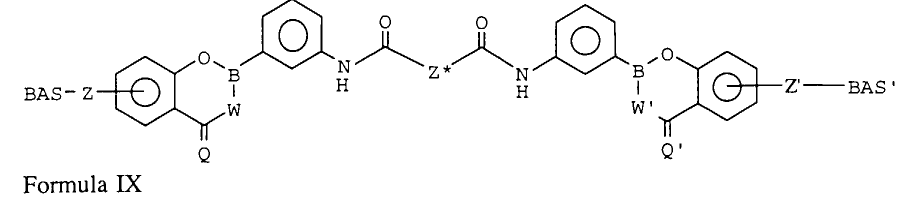 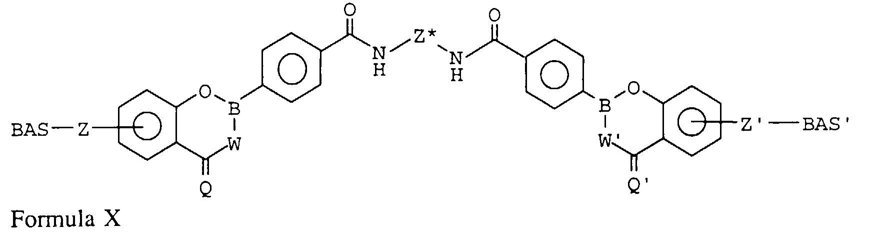 wherein
Q and Q' are independently selected from O, S, NH, N-alkyl, N-aryl and NCH₂-aryl ;
Y and Y' are independently selected from O, NH, N-alkyl, alkyl and aryl;
Z, Z', Z* and Z*' are spacers independently selected from alkyl and polyether chains, of from 1 to 16 carbon equivalents in length, wherein the chain may contain intermediate amide and disulfide bonds;
BAS, BAS', BAS^{*}, and BAS^{*}' are bioactive species, which may be the same or different;
X and X' are independently selected from H, CH₃ and C₆H₅;
W and W' are independently selected from either O, NH, N-alkyl, NC₆H₅, N-aryl, NCH₂-aryl, NCH₂CH₂OH, NCOCH₂CH₂OH, NOH, NO-alkyl and NOCH₂-aryl;
wherein unless otherwise specified, alkyl denotes a hydrocarbon moiety of up to six carbons, and aryl is selected from an aromatic ring, a substituted aromatic ring and fused aromatic rings.

3. Use of phenylboronic acid semiconjugates of formula XV 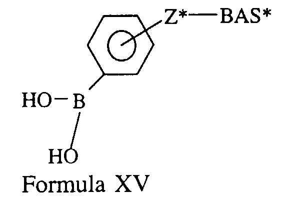 wherein Z* and BAS* are as defined in claim 2 in the production of bioconjugate complexes according to claim 1 or 2.

4. Phenylboronic acid complexing semiconjugate selected from formula XIII, XX and XXI 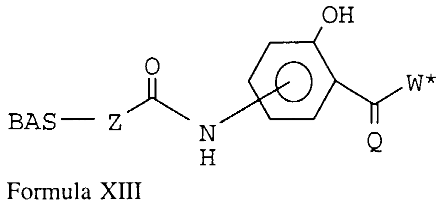 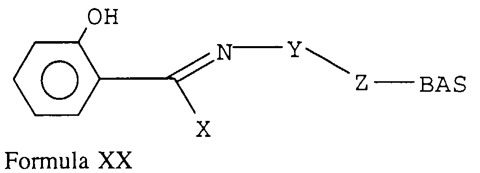 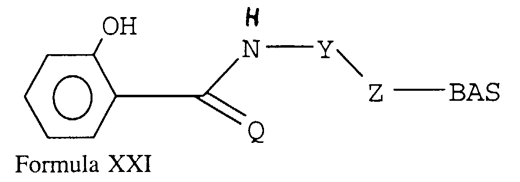 wherein Q, X, Y, Z, and BAS are as defined in claim 2 and W* is selected from H, OH, NH₂, NHCH₃, NHOH and NHOCH₃.

5. Use of phenylboronic acid reagents of formula XIV 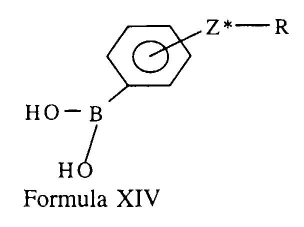 wherein Z^{*} is as defined in claim 2, and R is an electrophilic or nucleophilic moiety suitable for reaction with a bioactive species in the production of biological conjugates according to claim 1 or 2.

6. Phenylboronic acid complexing reagents selected from formulae XI, XVIII, and XIX 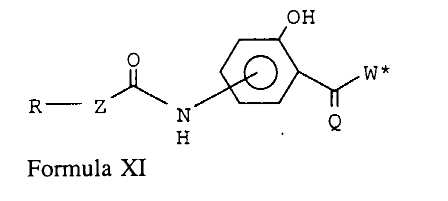 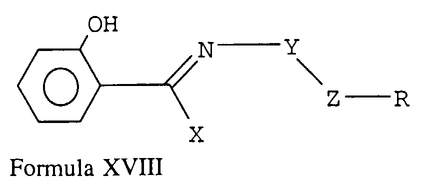 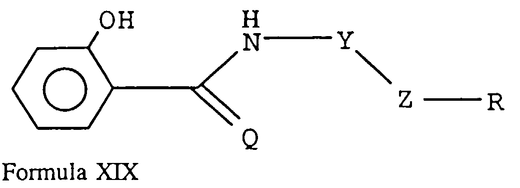 wherein Q, X, Y, and Z are as defined in claim 2. W* is as defined in claim 4, and R is an electrophilic or nucleophilic moiety suitable for reaction with a bioactive species.

7. Phenylboronic crosslinking reagents of formula XII 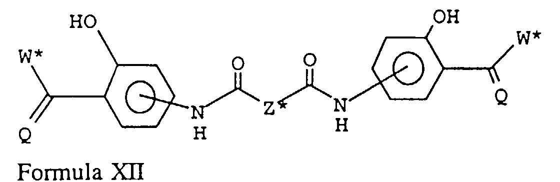 wherein, Q, and Z* are as defined in claim 2, and W* is as defined in claim 4.

8. Use of phenylboronic crosslinking reagents of formula XVI or XVII 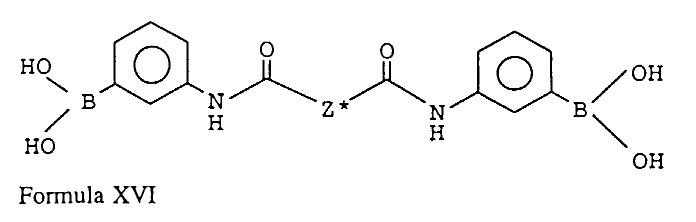 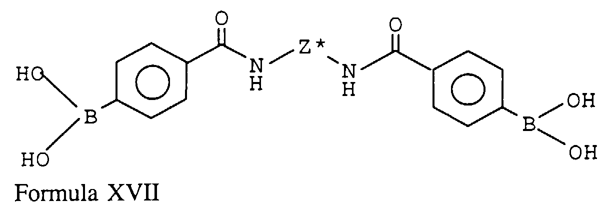 wherein Z* are as defined in claim 2 in the production of bioconjugate complexes according to claim 1 or 2.

9. Bioconjugate complexes or semiconjugates according to any one of claims 1 through 4 wherein at least one of the bioactive species is an antibody.

10. A kit or system for isolating a desired cell population comprising a bioconjugate or semiconjugate according to claim 9.

11. A process for isolating a desired cell population comprising the steps of contacting a medium containing cells with a bioconjugate complex according to claim 9, wherein the antibody recognises and binds to an epitope characterisic of the desired cell population, and separating the cells from the medium.

12. Bioconjugate complexes as claimed in claim 1, wherein E is derived from an analogue of salicylic acid.

## Patentansprüche

1. Biokonjugatkomplexe der Formel A
BAS-L-Bc-L'-(Bc'-L")ₙ-BAS' (A)
worin BAS und BAS' für bioaktive Einheiten stehen (die gleich oder verschieden sein können), L, L' und L" für Linker stehen (die gleich oder verschieden sein können), Bc und Bc' für Phenylborsäurekomplexe (die gleich oder verschieden sein können) der Formel D-E oder der Formel E-D stehen, worin D für einen Phenylborsäurerest steht und E für einen Phenylborsäurekomplexierungsrest steht, worin der Phenylborsäurekomplexierungsrest einen an das Bor des Phenylborsäurekomplexierungsrests gebundenen Stickstoff umfaßt und n für 0 oder 1 steht.

2. Biokonjugatkomplexe nach Anspruch 1, die aus den Formeln I bis X ausgewählt sind 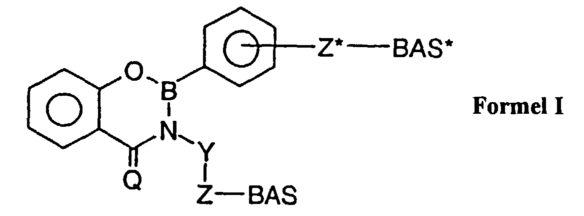 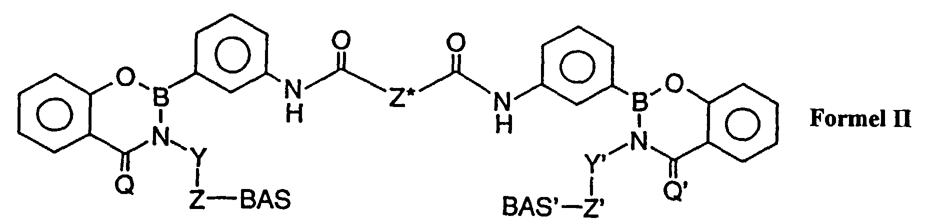 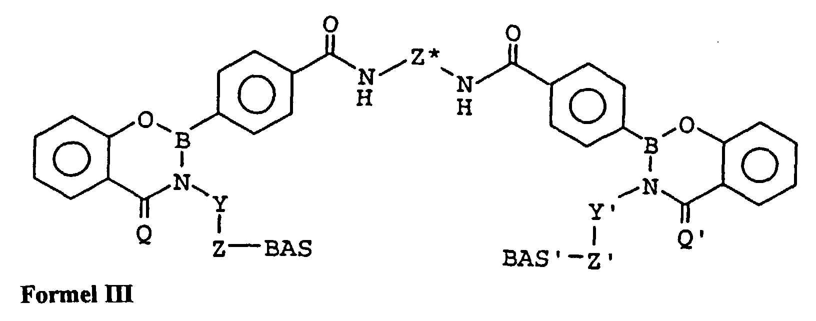 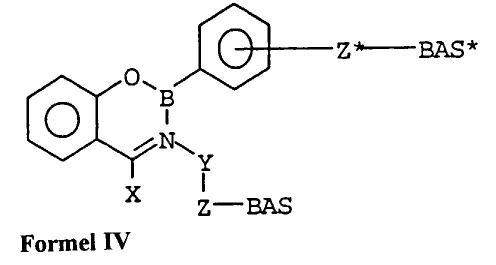 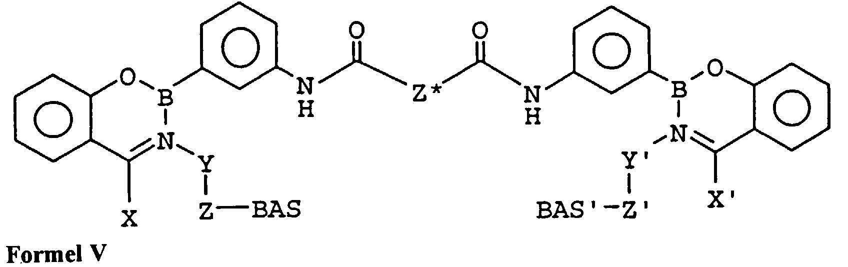 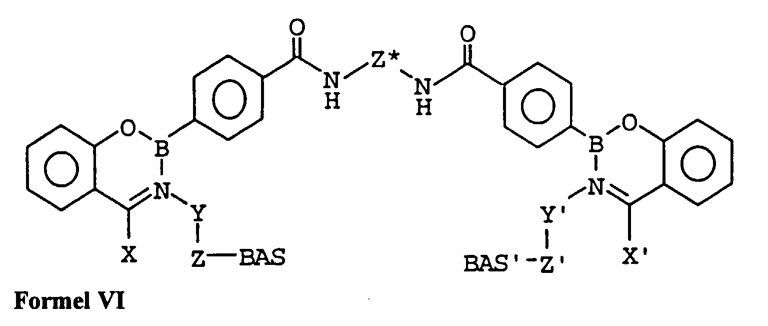 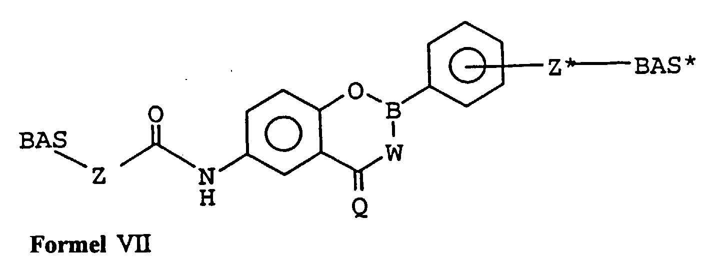 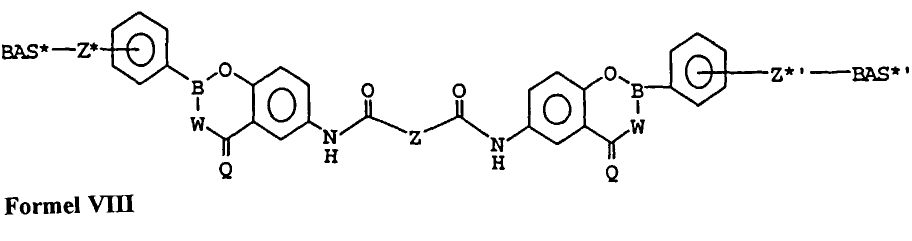 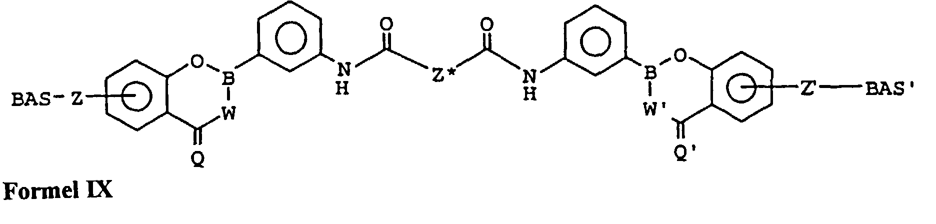 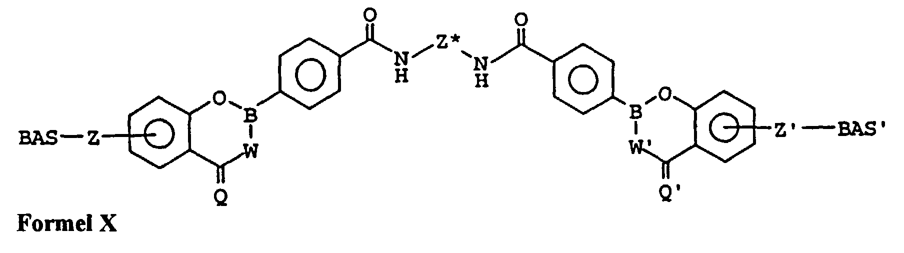 worin
Q und Q' unabhängig ausgewählt sind aus O, S, NH, N-Alkyl, N-Aryl und NCH₂-Aryl,
Y und Y' unabhängig ausgewählt sind aus O, NH, N-Alkyl, Alkyl und Aryl,
Z, Z', Z* und Z*' für Spacer stehen, die unabhängig ausgewählt sind aus Alkyl und Folyetherketten mit einer Länge von 1 bis 16 Kohlenstoffaquivalenten, worin die Kette Zwischenamide und Disulfidbindungen enthalten kann,
BAS, BAS', BAS* und BAS*' für bioaktive Einheiten stehen, die gleich oder verschieden sein können, X und X' unabhängig ausgewählt sind aus H, CH₃ und C₆H₅,
W und W' unabhängig ausgewählt sind aus O,NH,N-Alkyl,NC₆H₅, N-Aryl,NCH₂-Aryl,NCH₂CH₂OH, NCOCH₂CH₂OH, NOH, NO-Alkyl und NOCH₂-Aryl,
worin, falls nichts anderes angegeben ist, Alkyl für einen Kohlenwasserstoffrest mit bis zu sechs Kohlenstoffen steht und Aryl ausgewählt ist aus einem aromatischen Ring, einem substituierten aromatischen Ring und fusionierten aromatischen Ringen.

3. Verwendung von Phenylborsäuresemikonjugaten der Formel XV 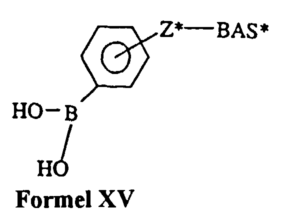 worin Z* und BAS* wie in Anspruch 2 definiert sind, zur Herstellung von Biokonjugatkomplexen nach Anspruch 1 oder 2.

4. Phenylborsäurekomplexierungssemikonjugat, ausgewählt aus der Formel XIII, XX und XXI 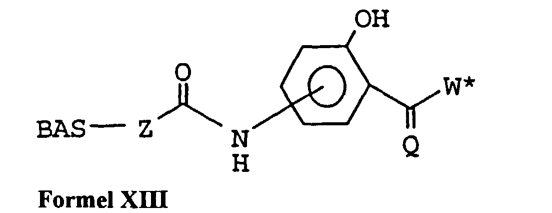 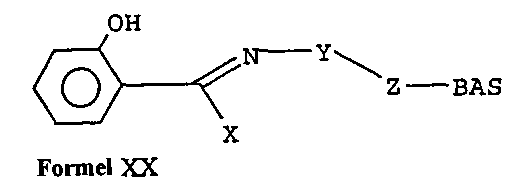 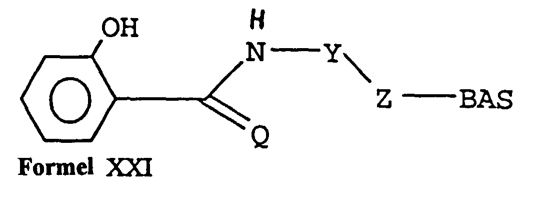 worin Q, X, Y, Z und BAS wie in Anspruch 2 definiert sind und W* ausgewählt ist aus H, OH, NH₂, NHCH₃, NHOH und NHOCH₃.

5. Verwendung von Phenylborsäurereagenzien der Formel XIV 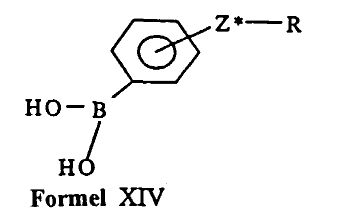 worin Z* wie in Anspruch 2 definiert ist und R für einen elektrophilen oder nukleophilen Rest steht, der zur Reaktion mit einer bioaktiven Einheit geeignet ist zur Herstellung von biologischen Konjugaten gemäß Anspruch 1 oder 2.

6. Phenylborsäurekomplexierungsreagenzien, die ausgewählt sind aus den Formeln XI, XVIII und XIX 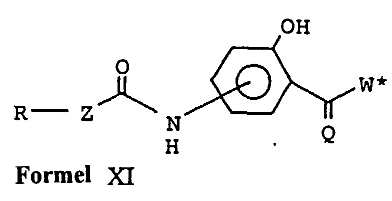 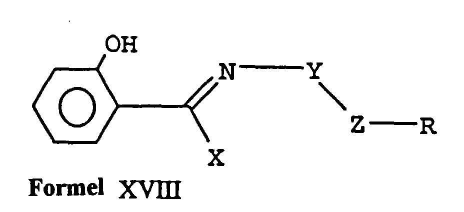 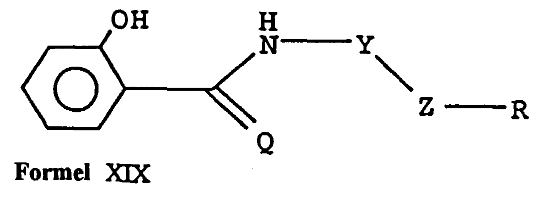 worin Q, X, Y und Z wie in Anspruch 2 definiert sind, W* wie in Anspruch 4 definiert ist und R für einen elektrophilen oder nukleophilen Rest steht, der zur Reaktion mit einer bioaktiven Einheit geeignet ist.

7. Phenylborsäurequervernetzungsreagenzien der Formel XII 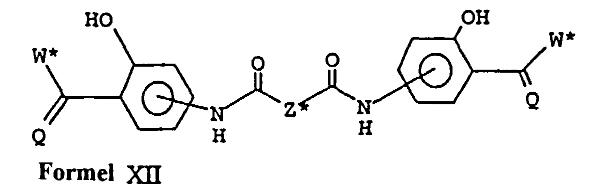 worin Q und Z* wie in Anspruch 2 definiert sind und W* wie in Anspruch 4 definiert ist.

8. Verwendung von Phenylborsäurequervernetzungsreagenzien der Formel XVI oder XVII 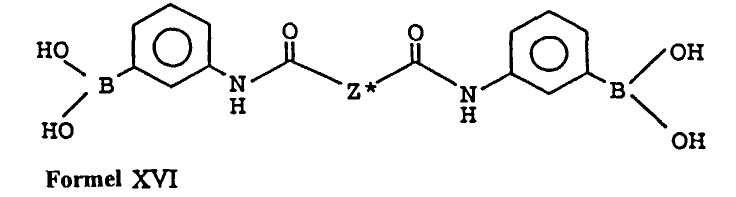 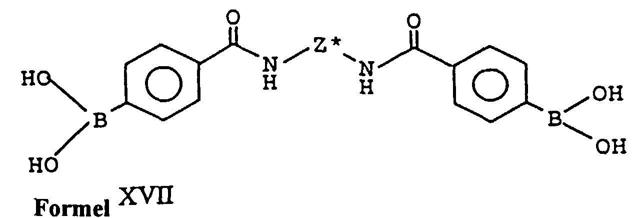 worin Z* wie in Anspruch 2 definiert ist zur Herstellung von Biokonjugatkomplexen nach Anspruch 1 oder 2.

9. Biokonjugatkomplexe oder Semikonjugate nach einem der Ansprüche 1 bis 4, worin zumindest eine der bioaktiven Einheiten ein Antikörper ist.

10. Kit oder System zur Isolierung einer gewünschten Zellpopulation, umfassend ein Biokonjugat oder Semikonjugat nach Anspruch 9.

11. Verfahren zur Isolierung einer gewünschten Zellpopulation, das die Schritte des Zusammenbringens eines Zellen enthaltenden Mediums mit einem Biokonjugatkomplex nach Anspruch 9, worin der Antikörper ein Epitop erkennt und bindet, das für die gewünschte Zellpopulation charakteristisch ist, und des Abtrennens der Zellen vom Medium umfaßt.

12. Biokonjugatkomplexe nach Anspruch 1, worin E von einem Analogon der Salicylsäure abgeleitet ist.

## Revendications

1. Des complexes de bioconjugués de formule A:
BAS-L-Bc-L'-(Bc'-L")ₙ-BAS' (A)
où BAS et BAS' sont des espèces bioactives (qui peuvent être identiques ou différentes); L, L' et L" sont des lieurs (qui peuvent être identiques ou différents); Bc et Bc' sont des complexes de l'acide phénylboronique (qui peuvent être identiques ou différents) de formule D-E ou de formule E-D où D signifie un reste d'acide phénylboronique et E signifie un reste complexant d'acide phénylboronique où le reste complexant d'acide phénylboronique inclut un azote lié au bore du reste complexant d'acide phénylboronique, et n signifie 0 ou 1.

2. Des complexes de bioconjugués tels que spécifiés à la revendication 1 choisis parmi les formules I à X: 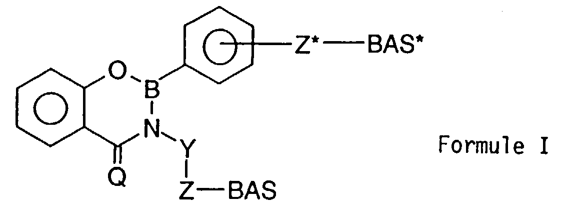 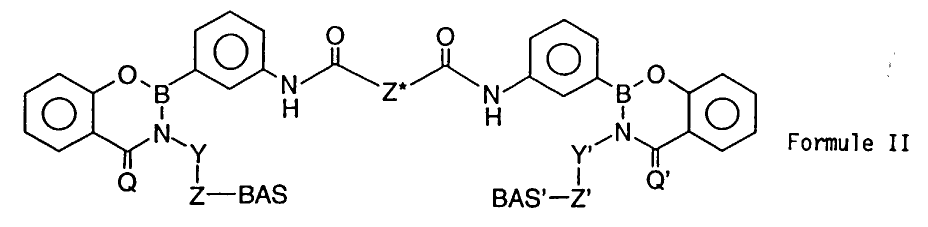 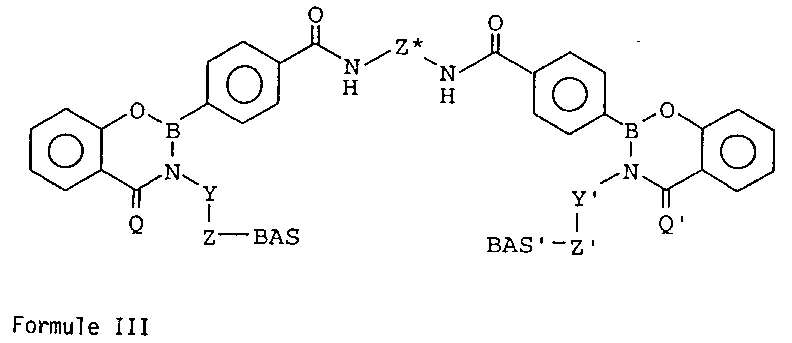 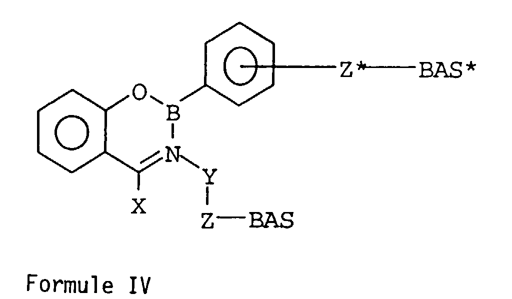 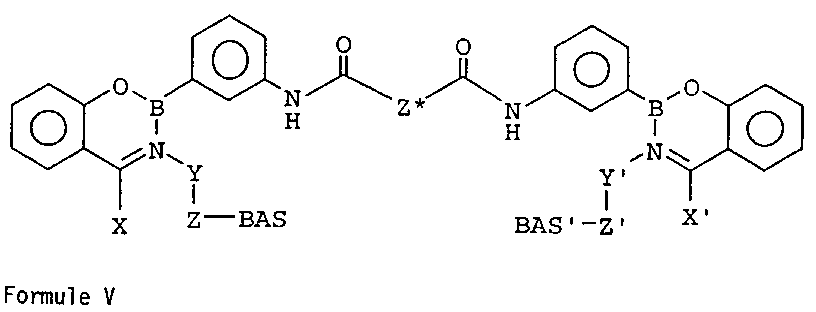 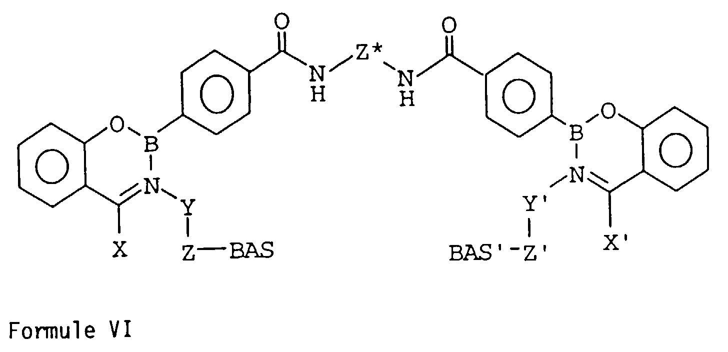 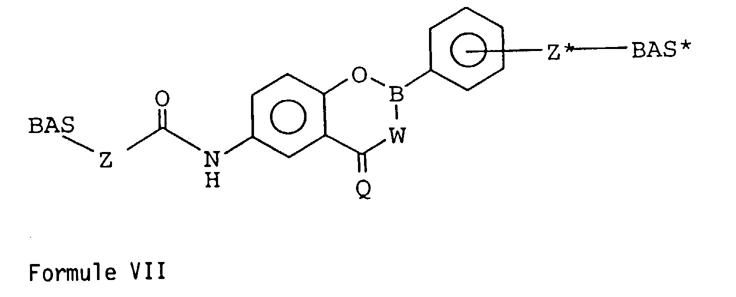 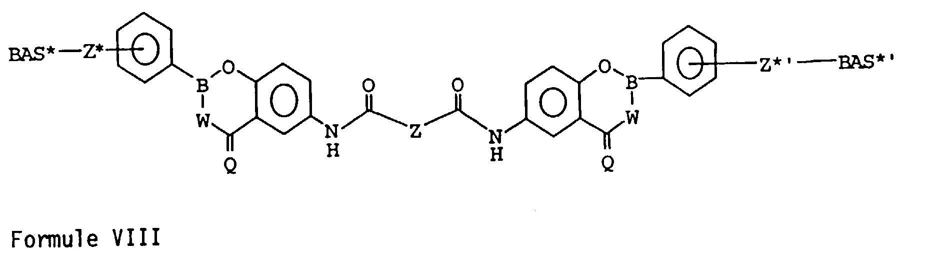 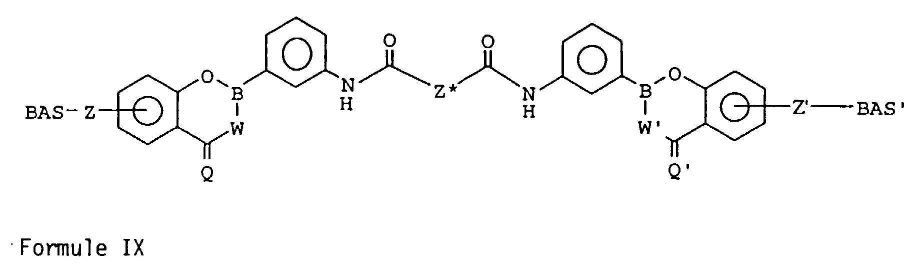 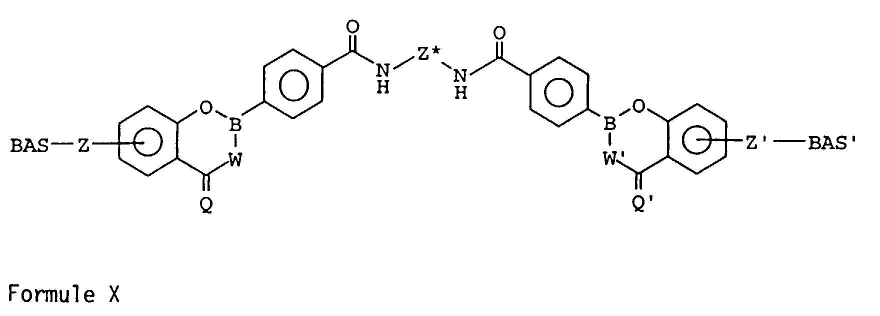 où
Q et Q' sont choisis indépendamment parmi O, S, NH, N-alkyle, N-aryle et NCH₂-aryle;
Y et Y' sont choisis indépendamment parmi O, NH, N-alkyle, alkyle et aryle;
Z, Z', Z* et Z*' sont des groupes espaceurs choisis indépendamment parmi les chaînes d'alkyle et de polyéthers ayant de I à 16 équivalents de carbone dans la longueur, où la chaîne peut contenir des liaisons intermédiaires d'amide et de disulfure;
BAS, BAS', BAS*, et BAS*' sont des espèces bioactives qui peuvent être identiques ou différentes;
X et X' sont choisis indépendamment parmi H, CH₃ et C₆H₅;
W et W' sont choisis indépendamment parmi O, NH, N-alkyle, NC₆H₅, N-aryle,
NCH₂-aryle, NCH₂CH₂OH, NCOCH₂CH₂OH, NOH, NO-alkyle et NOCH₂-aryle;
où, sauf indication contraire, alkyle signifie un reste hydrocarboné ayant jusqu'à 6 carbones, et aryle est choisi parmi un cycle aromatique, un cycle aromatique substitué et des cycles aromatiques condensés.

3. L'utilisation de semiconjugués de l'acide phénylboronique de formule XV 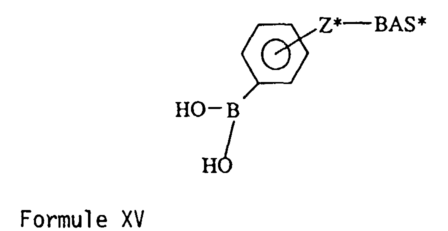 où Z* et BAS* sont tels que définis à la revendication 2, pour la production de complexes de bioconjugués selon la revendication 1 ou 2.

4. Un semiconjugué complexant de l'acide phénylboronique choisi parmi la formule XIII, XX et XXI 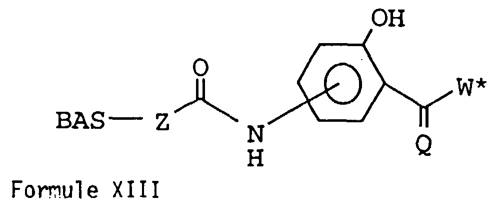 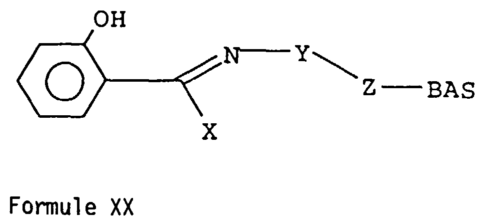 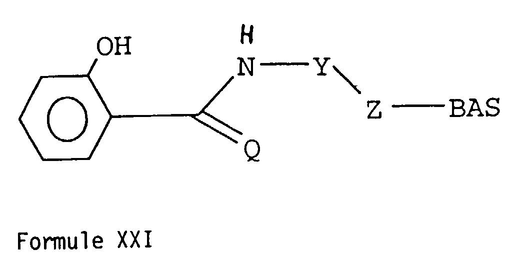 où Q, X, Y, Z et BAS sont tels que définis à la revendication 2 et W* est choisi parmi H, OH, NH₂, NHCH₃, NHOH et NHOCH₃.

5. L'utilisation de réactifs de l'acide phénylboronique de formule XIV 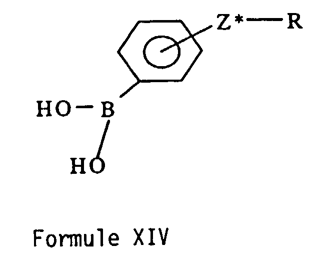 où Z* est tel que défini à la revendication 2, et R signifie un reste électrophile ou nucléophile approprié pour la réaction avec une espèce bioactive pour la production de conjugués biologiques selon la revendication 1 ou 2.

6. Des réactifs complexant de l'acide phénylboronique choisis parmi les formules XI, XVIII, et XIX 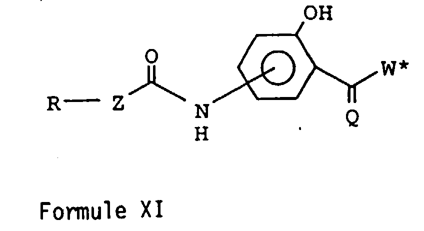 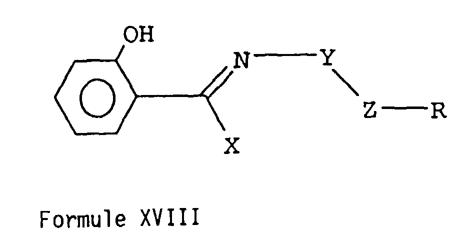 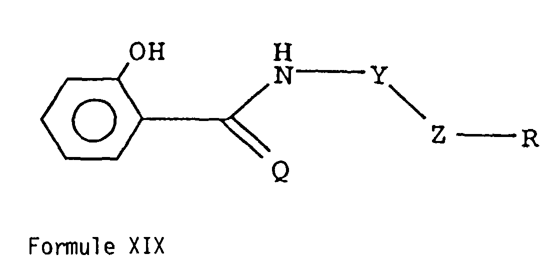 où Q, X, Y et Z sont tels que définis à la revendication 2, W* est tel que défini à la revendication 4, et R signifie un reste électrophile ou nucléophile approprié pour la réaction avec une espèce bioactive.

7. Des réactifs de réticulation phénylboroniques de formule XII 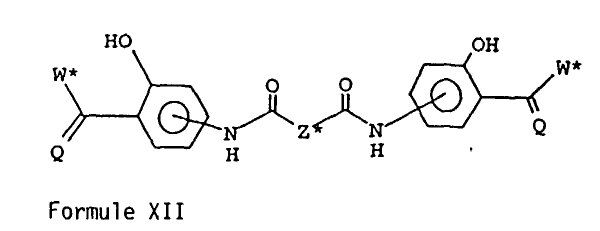 où Q, et Z* sont tels que définis à la revendication 2, et W* est tel que défini à la revendication 4.

8. L'utilisation de réactifs de réticulation phénylboroniques de formule XVI ou XVII 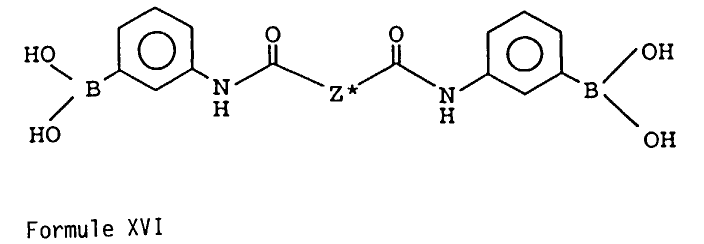 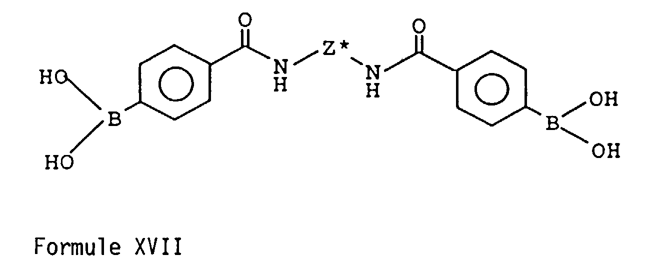 où Z* est tel que défini à la revendication 2 pour la production de complexes de bioconjugués selon la revendication 1 ou 2.

9. Des complexes de bioconjugués ou des semiconjugués selon l'une quelconque des revendications 1 à 4, où au moins une des espèces bioactives est un anticorps.

10. Un kit ou un système pour isoler une population cellulaire désirée comprenant un bioconjugué ou un semiconjugué selon la revendication 9.

11. Un procédé d'isolement d'une population cellulaire désirée comprenant les étapes de mise en contact d'un milieu contenant des cellules avec un complexe de bioconjugué selon la revendication 9, où l'anticorps reconnaît et se lie à un épitope caractéristique de la population cellulaire désirée, et de séparation des cellules du milieu.

12. Des complexes de bioconjugués tels que spécifiés à la revendication 1, où E est dérivé d'un analogue de l'acide salicylique.
